# EUROPEAN PATENT SPECIFICATION

(11) **EP 1 032 556 B1**
(45) Date of publication and mention of the grant of the patent: **19.12.2007**
(21) Application number: 98957349.8
(22) Date of filing: 20.10.1998
(51) Int. Cl.: C07C 233/00, C07C 235/00, C07C 237/00, C07D 207/08, C07D 207/30, C07D 207/46, C07D 209/18, C07D 211/30, C07D 211/32, C07D 215/38, C07D 233/66, C07D 233/90, C07D 239/02, C07D 241/00, C07D 241/02, C07D 249/04, C07D 249/08, C07D 249/14, C07D 261/06, C07D 263/34, C07D 265/30

(54) **PHARMACEUTICALLY ACTIVE COMPOUNDS AND METHODS OF USE**
PHARMAZEUTISCH WIRKSAME VERBINDUNGEN UND METHODEN ZU IHRER ANWENDUNG
COMPOSES PHARMACEUTIQUEMENT ACTIFS ET PROCEDES D'UTILISATION

(30) Priority: 21.10.1997 US 64830 P
(43) Date of publication of application: 06.09.2000
(73) Proprietor: Wyeth, Madison, NJ 07940 (US)
(72) Inventor: DURANT, Graham, J., Wellesley, MA 02181 (US); PADMANABHAN, Seetharamaiyer, Malden, MA 02148 (US)
(74) Representative: Plougmann & Vingtoft A/S
(86) International application number: PCT/US1998/022309
(87) International publication number: WO 1999/020599

(56) References cited:
- EP-A1- 0 062 844
- WO-A-95/20950
- DE-A1- 2 433 625
- DE-A1- 2 531 343
- DE-A1- 2 545 647
- DE-A1- 2 733 440
- DE-A1- 2 802 757
- FR-A1- 2 222 375
- US-A- 3 972 872
- US-A- 4 156 734
- MALYUGA, O. A. ET AL: "Antituberculous activity of guanidine derivatives" KHIMIKO-FARMATSEVTICHESKII ZHURNAL , 5(3), 12-16 CODEN: KHFZAN; ISSN: 0023-1134, 1971, XP008034606
- DATABASE CHEMABS [Online] CHEMICAL ABSTRACTS SERVICE, COLUMBUS, OHIO, US; MALYUGA, O. A. ET AL: "Antituberculous activity of guanidine derivatives" XP002293989 retrieved from STN Database accession no. 74:136938 & KHIMIKO-FARMATSEVTICHESKII ZHURNAL , 5(3), 12-16 CODEN: KHFZAN; ISSN: 0023-1134, 1971,
- NAKAYAMA et al., "Synthesis and Structure-Activity Study of Protease Inhibitors. V. Chemical Modification of 6-Amidino-2-Naphthyl 4-Guanidinobenzoate", CHEMICAL PHARMACEUTICAL BULLETIN, January 1993, Vol. 41, No. 1, pages 117-125, XP002106226.
- OKAJIMA et al., "Synthesis and Reaction of 2-Imino-1,3-Thiazetidines and 2-Imino-1,3-Dithietanes", JOURNAL OF HETEROCYCLIC CHEMISTRY, January 1991, Vol. 28, No. 1, pages 177-185, XP002920278
- BUSCEMI et al., "Heterocyclic Photorearrangements. Photochemical Behaviour of Some 3,4-Disubstituted 1,2,4- Oxadiazoles in Methanol at 254 nm", JOURNAL OF HETEROCYCLIC CHEMISTRY, May-June 1988, Vol. 25, No. 3, pages 931-935, XP002920279
- BUSCHAUER V.A., "Synthese und Pharmakologische Wirkung von Arylmethylthioethylguanidinen", ARZNEIMITTEL FORSCHUNG, DRUG RES. September 1987, Vol. 37, No. 9, pages 1008-1012, XP002920280
- TRUMM et al., "Tetrahydroisochinoline als Bausteine von H2-Antagonisten", ARZNEIMITTEL FORSCHUNG, DURG RES., August 1986, Vol. 36, No. 8, pages 1169-1174, XP002920281
- SCHNELLER et al., "Synthesis of Proximal-Benzoguanine and a Simplified Synthesis of Proximal-Benzohypoxanthine", JOURNAL OF ORGANIC CHEMISTRY, 1986, Vol. 51, No. 21, pages 4067-4070, XP002920282
- NAGASAWA et al., "Alkylation of Thioureas and Related Compounds by use of Alcohols, Diethyl Azodicarboxylate and Triphenylphosphine", THE CHEMICAL SOCIETY OF JAPAN, BULL. CHEM. SOC. JPN., July 1981, Vol. 54, No. 7, pages 2223-2224, XP002920283
- MITSUNOBU et al., "Preparations and Reactions of N-Benzoyl- and N-Ethoxycarbonylcarbodiimides", BULLETIN OF THE CHEMICAL SOCIETY OF JAPAN, December 1972, Vol. 45, No. 12, pages 3607-3611, XP002920284
- GUND et al., "A Novel Reaction of Guanidine with Benzaldehydes", TETRAHEDRON LETTERS, September 1972, No. 38, pages 3983-3986, XP002920285
- YAMAZAKI et al., "Synthesis of Guanosine and its Derivatives from 5-Amino-1-beta-D-Ribofuranosyl-1-Imidazolec arboxamide. I. Ring Closure with Benzoyl Isothioeyanate¹", JOURNAL OF ORGANIC CHEMISTRY, June 1967, Vol. 32, No. 6, pages 1825-1828, XP002920286

## Description

### CROSS-REFERENCE TO RELATED APPLICATIONS

The present application is a continuation-in-part of U.S. provisional application 60/064,830, filed October 21, 1997, which application is incorporated herein by reference in its entirety.

### BACKGROUND OF THE INVENTION

### 1. Field of the Invention

The present invention relates to pharmaceutically active compounds, including acylguanidine compounds, and uses and pharmaceutical compositions that utilize or comprise one or more such compounds. Compounds of the invention are particularly useful for the treatment or prophylaxis of neurological injury and neurodegenerative disorders.

### 2. Background

Nerve cell death (degeneration) can cause potentially devastating and irreversible effects for an individual and may occur e.g. as a result of stroke, heart attack or other brain or spinal chord ischemia or trauma. Additionally, neurodegenerative disorders involve nerve cell death (degeneration) such as Alzheimer's disease, Parkinson's disease, Huntington's disease, Amyotrophic Lateral Sclerosis, Down's Syndrome and Korsakoffs disease.

Therapies have been investigated to treat nerve cell degeneration and related disorders, e.g., by limiting the extent of nerve cell death that may otherwise occur to an individual. See, e.g., N. L. Reddy et al., J. Med. Chem., 37:260-267 (1994); and WO 95/20950.

The compound MK-801 has exhibited good results in a variety of *in vivo* models of stroke. See B. Meldrum, Cerbrovascular Brain Metab. Rev., 2:27-57 (1990); D. Choi, Cerbrovascular Brain Metab. Rev., 2:105-147 (1990). See also Merck Index, monograph 3392, 11th ed., 1989. For example, MK-801 exhibits good activity in mouse audiogenic tests, a recognized model for evaluation of neuroprotective drugs. See, e.g., M. Tricklebank et al., European Journal of Pharmacology, 167:127-135 (1989); T. Seyfried, Federation Proceedings, 38(10):2399-2404 (1979).

However, MK-801 also has shown toxicity and further clinical development of the compound is currently uncertain. See J. W. Olney et al., Science, 244:1360-1362 (1989); W. Koek et al., J. Pharmacol. Exp. Ther., 252:349-357 (1990); F.R. Sharp et al., Society for Neuroscience Abstr., abstr. no. 482.3 (1992).

It thus would be highly desirable to have new neuroprotective agents, particularly agents to limit the extent or otherwise treat nerve cell death (degeneration) such as may occur with stroke, heart attack or brain or spinal cord trauma, or to treat neurodegenerative disorders such as Alzheimer's disease, Parkinson's disease, Huntington's disease, Amyotrophic Lateral Sclerosis, Down's Syndrome and Korsakoffs disease.

### SUMMARY OF THE INVENTION

In a first aspect, the present invention provides acylguanidine compounds selected from :
N-(4-methylbenzoyl)-N'-methyl-N'-(3-methylthiophenyl)guanidine;
N-(4-methylbenzoyl)-N'-methyl-N'-(3-iodophenyl)guanidine;
N-(4-methylbenzoyl)-N'-(1-naphthyl)guanidine;
N-(4-methylbenzoyl)-N'-(4-benzyloxyphenyl)guanidine;
N-(4-methylbenzoyl)-N'-(4-tertbutylphenyl)guanidine;
N-(4-methylbenzoyl)-N'-(4-isopropylphenyl)guanidine;
N-(4-methylbenzoyl)-N'-(2,5-dibromophenyl)guanidine;
N-(4-methylbenzoyl)-N'-(4-isopropoxyphenyl)guanidine;
N-(4-methylbenzoyl)-N'-(3,4,5-trimethoxyphenyl)guanidine;
N-(4-methylbenzoyl)-N'-(2-isopropylphenyl)guanidine;
N-(2,5-dichlorobenzoyl)-N'-methyl-N'-(3-iodophenyl)guanidine;
N-(2,5-dichlorobenzoyl)- N'-methyl-N'-(3-methylthiophenyl)guanidine;
N-(2,5-dichlorobenzoyl)-N'-(1-naphthyl)guanidine;
N-(2,5-dichlorobenzoyl)-N'-(4-benzyloxyphenyl)guanidine;
N-(2,5-dichlorobenzoyl)-N'-(4-isopropylphenyl)guanidine;
N-(2,5-dichlorobenzoyl)-N'-(4-tertbutylphenyl)guanidine;
N-(2,5-dichlorobenzoyl)-N'-methyl-N'-(4-isopropylphenyl)guanidine;
N-(phenylacetyl)-N'-(4-benzyloxyphenyl)guanidine;
N-(phenylacetyl)-N'-(4-isopropylphenyl)guanidine;
N-(phenylacetyl)-N'-(4-tert-butylphenyl)guanidine;
N-(phenylacetyl)-N'-(4-isopropoxyphenyl)guanidine;
N-(phenylacetyl)-N'-(4-isopropylphenyl)-N'-methylguanidine;
N-(adamantan-1-carbonyl)-N'-methyl-N'-(3-iodophenyl)guanidine;
N-(adamantan-1-carbonyl)-N'-(1-naphthyl)guanidine;
N-(adamantan-1-carbonyl)-N'-(4-benzyloxyphenyl)guanidine;
N-(adamantan-1-carbonyl)-N'-(4-isopropylphenyl)guanidine;
N-(adamantan-1-carbonyl)-N'-(4-tert-butylphenyl)guanidine;
N-(adamantan-1-carbonyl)-N'-(2,5-dibromophenyl)guanidine;
N-(adamantan-1-carbonyl)-N'-(4-isopropylphenyl)-N'-methyl)guanidine;
N-(4-chlorobenzoyl)-N'-methyl-N'-(3-iodophenyl)guanidine;
N-(4-chlorobenzoyl)-N'-methyl-N'-(3-methylthiophenyl)guanidine;
N-(4-chlorobenzoyl)-N'-(1-naphthyl)guanidine;
N-(4-chlorobenzoyl)-N'-(4-benzyloxyphenyl)guanidine;
N-(4-chlorobenzoyl)-N'-(4-isopropylphenyl)guanidine;
N-(4-chlorobenzoyl)-N'-(4-tert-butylphenyl)guanidine;
N-(4-chlorobenzoyl)-N'-(2,5-dibromophenyl)guanidine;
N-(3,4-dichlorobenzoyl)-N'-methyl-N'-(3-iodophenyl)guanidine;
N-(3,4-d(chlorobenzoyl)-N'-(1-naphthyl)guanidine;
N-(3,4-dichlorobenzoyl)-N'-(4-benzyloxyphenyl)guanidine;
N-(3,4-dichlorobenzoyl)-N'-(4-isopropylphenyl)guanidine;
N-(3,4-dichlorobenzoyl)-N'-(4-tert-butylphenyl)guanidine;
N-(3,4-dichlorobenzoyl)-N'-methyl-N'-(4-isopropylphenyl)guanidine;
N-(thiophen-2-carbonyl)-N'-methyl-N'-(3-iodophenyl)guanidine;
N-(thiophen-2-carbonyl)-N'-methyl-N'-(3-methylthiophenyl)guanidine;
N-(thiophen-2-carbonyl)-N'-(1-naphthyl)guanidine;
N-(thiophen-2-carbonyl)-N'-(4-benzyloxyphenyl)guanidine;
N-(thiophen-2-carbonyl)-N'-(4-isopropylphenyl)guanidine;
N-(thiophen-2-carbonyl)-N'-(4-tert-butylphenyl)guanidine;
N-(thiophen-2-carbonyl)-N'-methyl-N'-(4-isopropylphenyl)guanidine;
N-(furan-2-carbonyl)-N'-methyl-N'-(3-iodophenyl)guanidine;
N-(furan-2-carbonyl)-N'-methyl-N'-(3-methylthiophenyl)guanidine;
N-(furan-2-carbonyl)-N'-(1-naphthyl)guanidine;
N-(furan-2-carbonyl)-N'-(4-benzyloxyphenyl)guanidine;
N-(furan-2-carbonyl)-N'-(4-isopropylphenyl)guanidine;
N-(furan-2-carbonyl)-N'-(4-tert-butylphenyl)guanidine;
N-(furan-2-carbonyl)-N'-(4-isopropylphenyl)-N'-methylguanidine;
N-(pyridin-3-carbonyl)-N'-(1-naphthyl)guanidine;
N-(pyridin-3-carbonyl)-N'-(4-benzyloxyphenyl)guanidine;
N-(pyridin-3-carbonyl)-N'-(4-isopropylphenyl)guanidine;
N-(pyridin-3-carbonyl)-N'-(4-tert-butylphenyl)guanidine;
N-(4-methoxybenzoyl)-N'-(4-benzyloxyphenyl)guanidine;
N-(4-methoxybenzoyl)-N'-(4-isopropylphenyl)guanidine;
N-(4-methoxybenzoyl)-N'-(4-isopropoxyphenyl)guanidine;
N-(4-methoxybenzoyl)-N'-(3,4,5-trimethoxyphenyl)guanidine;
N-(1-naphthoyl)-N'-(4-benzyloxyphenyl)guanidine;
N-(1-naphthoyl)-N'-(4-isopropylphenyl)guanidine;
N-(1-naphthoyl)-N'-(4-isopropoxyphenyl)guanidine;
N-(3,4,5-trimethoxybenzoyl)-N'-(2-isopropylphenyl)guanidine;
N-(3,4,5-trimethoxybenzoyl)-N'-(4-isopropoxyphenyl)guanidine;
N-(4-butoxybenzoyl)-N'-(2-isopropylphenyl)guanidine;
N-(4-butoxybenzoyl)-N'-(4-isopropoxyphenyl)guanidine;
N-(4-butoxybenzoyl)-N'-(3,4,5-trimethoxyphenyl)guanidine;
N-(4-ethoxybenzoyl)-N'-(2-isopropylphenyl)guanidine;
N-(4-ethoxybenzoyl)-N'-(4-isopropoxyphenyl)guanidine;
N-(4-methylbenzoyl)-N'-(benzyl)guanidine;
N-(4-methylbenzoyl)-N'-(2-phenethyl)guanidine;
N-(4-methylbenzoyl)-N'-(4-phenylbutyl)guanidine;
N-(4-methylbenzoyl)-N'-(3-phenylpropyl)guanidine;
N-(4-methylbenzoyl)-N'-(1-naphthylmethyl)guanidine;
N-(4-methylbenzoyl)-N'-(2-(4-chlorophenyl)ethyl)guanidine;
N-(4-methylbenzoyl)-N'-(5-phenylpentyl)guanidine;
N-(4-methylbenzoyl)-N'-(3-phenoxypropyl)guanidine;
N-(3,4-dichlorobenzoyl)-N'-(benzyl)guanidine;
N-(3,4-dichlorobenzoyl)-N'-(3-phenylpropyl)guanidine;
N-(4-chlorobenzoyl)-N'-(2-phenethyl)guanidine;
N-(4-chlorobenzoyl)-N'-(4-phenylbutyl)guanidine;
N-(4-methoxybenzoyl)-N'-(2-phenethyl)guanidine;
N-(4-methoxybenzoyl)-N'-(4-phenylbutyl)guanidine;
N-(4-methoxybenzoyl)-N'-(2-(4-chlorophenylethyl)guanidine;
N-(4-methoxybenzoyl)-N'-(1-naphthylmethyl)guanidine;
N-(4-methoxybenzoyl)-N'-(3,4,5-trimethoxybenzyl)guanidine;
N-(4-ethoxybenzoyl)-N'-(4-phenylbutyl)guanidine;
N-(4-ethoxybenzoyl)-N'-(2-phenethyl)guanidine;
N-(4-ethoxybenzoyl)-N'-(2-(4-chlorophenyl)ethyl)guanidine;
N-(4-ethoxybenzoyl)-N'-(3-phenylpropyl)guanidine;
N-(4-ethoxybenzoyl)-N'-(1-naphthylmethyl)guanidine;
N-(4-butoxybenzoyl)-N'-(4-phenylbutyl)guanidine;
N-(4-butoxybenzoyl)-N'-(2-phenethyl)guanidine;
N-(4-butoxybenzoyl)-N'-(2-(4-chlorophenyl)ethyl)guanidine;
N-(4-butoxybenzoyl)-N'-(3-phenylpropyl)guanidine;
N-(4-butoxybenzoyl)-N'-(2-(3-indole)ethyl)guanidine;
N-(3,4,5-trimethoxybenzoyl)-N'-(4-phenylbutyl)guanidine;
N-(3,4,5-trimethoxybenzoyl)-N'-(2-(3-indole)ethyl)guanidine;
N-(3,4,5-trimethoxybenzoyl)-N'-(2-phenylethyl)guanidine;
N-(1-naphthoyl)-N'-(benzyl)guanidine;
N-(1-naphthoyl)-N'-(2-phenylethyl)guanidine;
N-(1-naphthoyl)-N'-(4-phenylbutyl)guanidine;
N-(thiophen-2-carbonyl)-N'-(benzyl)guanidine;
N-(thiophen-2-carbonyl)-N'-(2-phenylethyl)guanidine;
N-(thiophen-2-carbonyl)-N'-(4-phenylbutyl)guanidine;
N-(4-methylbenzoyl)-N'-(cyclohexyl)-N"-methylguanidine;
N-(4-methylbenzoyl)-N'-(4-phenylbutyl)-N"-methylguanidine;
N-(4-methoxybenzoyl)-N'-(5-phenylpentyl)guanidine;
N-(2-methylbenzoyl)-N'-(4-phenylbutyl)guanidine;
N-(2-methylbenzoyl)-N'-(2-isopropylphenyl)guanidine;
N-(2-methylbenzoyl)-N'-(4-isopropylphenyl)guanidine;
N-(2-methylbenzoyl)-N'-(3-phenylpropyl)guanidine;
N-(4-methoxybenzoyl)-N'-(2-phenoxypropyl)guanidine;
N-(4-butoxybenzoyl)-N'-(5-phenylpentyl)guanidine;
N-(4-methylbenzoyl)-N'-(2-phenoxyethyl)guanidine;
N-(4-methoxybenzoyl)-N'-(2-phenoxyethyl)guanidine;
N-(4-ethoxybenzoyl)-N'-[(2-benzylthio)ethyl]guanidine;
N-(4-ethoxybenzoyl)-N'-(3,4,5-trimethoxyphenyl)guanidine;
or a pharmaceutically acceptable salt of any of said compounds.

Compounds of the invention are useful for a number of therapeutic applications. In particular, the invention includes methods for treatment and/or prophylaxis of neurological conditions/injuries such as epilepsy, neurodegenerative conditions and/or nerve cell death (degeneration) resulting from or associated with e.g. hypoxia, hypoglycemia, brain or spinal chord ischemia, retinal ischemia, brain or spinal chord trauma or post-surgical neurological deficits and the like as well as neuropathic pain. The invention also includes methods for treating peripheral necropathy. The compounds of the invention are especially useful for treatment of a person susceptible or suffering from stroke or heart attack or neurological deficits relating to cardiac arrest, a person suffering or susceptible to brain or spinal cord injury, or a person suffering from the effects of retinal ischemica or degeneration, or a person suffering from decreased blood flow or nutrient supply to retinal tissue or optic nerve, retinal trauma, glaucoma or optic nerve injury. Compounds of the invention also are useful to treat and/or prevent various neurodegenerative diseases such as Parkinson's disease, Huntington's disease, Amyotrophic Lateral Sclerosis, Alzheimer's disease, Down's Syndrome, Korsakoffs disease, cerebral palsy and/or age-dependent dementia. Compounds of the invention will be further useful to treat and/or prevent migraines, shingles (herpes zoster), epilepsy, emesis and/or narcotic withdrawal symptoms. Compounds of the invention will be useful for treatment of various types of pain, including e.g. chronic pain. The treatment methods of the invention in general comprise administration of a therapeutically effective amount of one or more compounds of the invention to an animal, including a mammal, particularly a human. Particularly preferred compounds of the invention exhibit good activity in an anticonvulsant *in vivo* mouse audiogenic assay e.g. as disclosed in Example 11which follows, preferably about 20% or more inhibition at a dose of a compound of the invention of 20 mg/kg, more preferably about 50% or more inhibition at a dose of 20 mg/kg in such an anticonvulsant *in vivo* audiogenic assay.

The invention also provides pharmaceutical compositions that comprise one or more compounds of the invention and a suitable carrier for the compositions.

Other aspects of the invention are disclosed *infra.*

### DETAILED DESCRIPTION OF THE INVENTION

We have now discovered that compounds of the invention are useful for therapeutic applications, including to treat neurological injury or a neurodegenerative disorder.

Compounds of the invention can be suitably prepared by one or more of several routes which are generally depicted in the following Scheme.

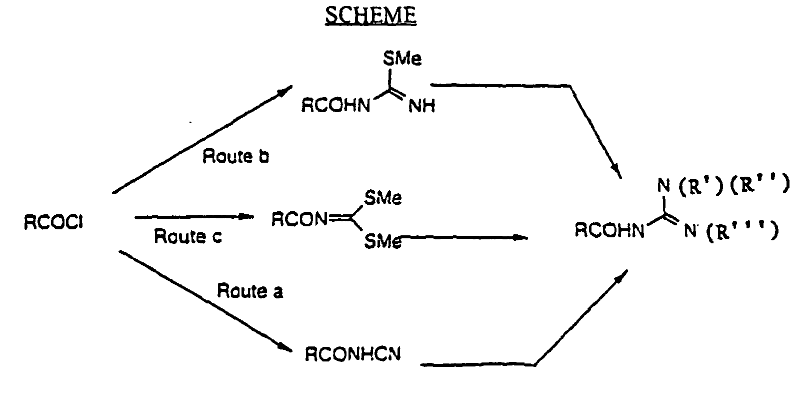

More specifically, as generally depicted in "Route a" above, compounds of the invention can be prepared by reaction of a suitable amine precursor compound with a suitable substituted cyanamide compound that provides the desired R group.

A secondary amine can be employed to prepare compounds where R² is other than hydrogen. For example, an N-methylaniline or other N-alkylaniline can be employed to provide an alkyl R² substituent.

Suitable cyanamide compounds will include aryloylcyanamide compounds (i.e. aryl(C=O)NHCN) such as substituted or unsubstituted benzoylcyanamide and the like; an arylalkanoyl cyanamide such as substituted or unsubstituted phenylacetylcyanamide (C₆H₅COCH₂NHCN) and the like; a cyclic alkanoyl cyanamide such as admantancarbonylcyanamide and the like; or a heteroaromatic(carbonyl)cyanamide or a heteroalicyclic(carbonyl)cyanamide such as (2-thiophenecarbonyl)cyanamide, (2- furanylcarbonyl)cyanamide, (3-pyridylcarbonyl)cyanamide, and the like; or an aralkyl, heteroaryl alkyl or heteroalicyclic alkyl reagant such as e.g. methyl phenylacetate and the like. See the examples which follow.

The cyanamide reactants can be readily prepared, e.g. by reaction of the corresponding substituted carbonylchloride reactant with cyanamide under suitable conditions, e.g. in the presence of base with stirring at room temperature until reaction completion. The reaction solution with the thus formed substituted cyanamide then can be neutralized and the product isolated by standard procedures. See the Examples which follow for exemplary conditions.

Typically a salt (e.g. an HCl salt) of the amine precursor compound is reacted with the substituted cyanamide reagent. The amine precursor can be reacted with the substituted cyanamide reagent in a suitable solvent such as chlorobenzene, toluene or xylene with heating (e.g. reflux temperature) until reaction completion, e.g. 2 or more hours.

As generally depicted in "Route b" above, compounds of the invention also can be prepared by reaction of a substituted carbonylisothiourea (RCONHC(=NH)SCH₃ in above Route b) and a substituted amine precursor compound, typicially a substituted aliphatic amine such as a arylalkylamine, e.g. NH₂CH₂CH₂CH₂CH₂C₆H₅. This "Route b" is particularly suitable for synthesis of compounds containing an alkylene group.

The isothiourea reagent can be readily prepared by reaction of S-methylisothiourea with a desired substituted carbonyl chloride such as a benzoyl chloride compound in the presence of base and in a suitable solvent such as aqueous diethyl ether at room temperature with stirring overnight. For exemplary conditions, see Example 5, Part I; Example 6, Part I; Example 7, Part I; and Example 8, Part I, which follow. The thus formed isothiourea derivative is then reacted with the substituted amine in the presence of base such as triethylamine in a suitable solvent such as toluene, chlorobenzene or other aromatic solvent with heating, e.g. reflux temperature. See Example 5, Part I; Example 6, Part I; Example 7, Part I; and Example 8, Part I, which follow for exemplary conditions.

As generally depicted in "Route c" above, compounds of the invention that contain an N"-substituent that is other than hydrogen can be suitably prepared by reaction of a substituted carbimidodithiolate (RCON=C(SCH₃)₂ in above Route c) and sequential reactions of substituted amine precursor compounds. The substituted carbimidodithiolate can be prepared by reaction of a desired substituted amide compound with carbon disulfide in a suitable solvent such as tetrahydrofuran and in the presence of base such as sodium hydride. The isolated substituted carbimidodithiolate compound is reacted with a slight molar excess of a desired substituted amine (provides the desired R group). The resultant thiourea is further reacted with a substituted amine to provide N" substitution. See Example 9 which follows for exemplary conditions.

As discussed above, the present invention includes methods for treating preventing certain neurological disorders, including the consequences of stroke, heart attack and traumatic head or brain injury, epilepsy or neurodegenerative diseases comprising the administration of an effective amount of one or more compounds of the invention to a subject including a mammal, such as a primate, especially a human, in need of such treatment. In particular, the invention provides methods for treatment and/or prophylaxis of nerve cell death (degeneration) resulting e.g. from hypoxia, hypoglycemia, brain or spinal cord ischemia, brain or spinal cord trauma, stroke, heart attack or drowning. Typical candidates for treatment include e.g. heart attack, stroke and/or persons suffering from cardiac arrest neurological deficits, brain or spinal cord injury patients, patients undergoing major surgery such as heart surgery where brain ischemia is a potential complication and patients such as divers suffering from decompression sickness due to gas emboli in the blood stream. Candidates for treatment also will include those patients undergoing a surgical procedure involving extra-corporal circulation such as e.g. a bypass procedure. Subjects suffering from or susceptible to peripheral necropathy can be treated in accordance with the invention by administration of an effective amount of one or more compounds of Formulae I through X or Formulae I' through X'.

The invention in particular provides methods for treatment which comprise administration of one or more compounds of the invention to a patient that is undergoing surgery or other procedure where brain or spinal cord ischemia is a potential risk. For example, carotid endarterectomy is a surgical procedure employed to correct atherosclerosis of the carotid arteries. Major risks associated with the procedure include intraoperative embolization and the danger of hypertension in the brain following increased cerebral blood flow, which may result in aneurism or hemorrhage. Thus, an effective amount of one or more compounds of the present invention could be administered pre-operatively or peri-operatively to reduce such risks associated with carotid endarterectomy, or other post-surgical neuorological deficits.

The invention further includes methods for prophylaxis against neurological deficits resulting from e.g. coronary artery bypass graft surgery and aortic valve replacement surgery, or other procedure involving extra-corporal circulation. Those methods will comprise administering to a patient undergoing such surgical procedures an effective amount of one or more compounds of the invention, typically either pre-operatively or peri-operatively.

The invention also provides methods for prophylaxis and treatment against neurological injury for patients undergoing myocardial infarction, a procedure that can result in ischemic insult to the patient. Such methods will comprise administering to a patient undergoing such surgical procedure an effective amount of one or more compounds of the invention, typically either pre-operatively or peri-operatively.

Also provided are methods for treating or preventing neuropathic pain such as may experienced by cancer patients, persons having diabetes, amputees and other persons who may experience neuropathic pain. These methods for treatment comprise administration of an effective amount of one or more compounds of the invention to a patient in need of such treatment.

The invention also provides methods for treatment and prophylaxis against retinal ischemia or degeneration and resulting visual loss. For example, a compound of the invention can be administered parenterally or by other procedure as described herein to a subject a suffering from or susceptible to ischemic insult that may adversely affect retinal function, e.g., significantly elevated intraocular pressures, diseases such as retinal artery or vein occlusion, diabetes or other ischemic ocular-related diseases. Post-ischemic administration also may limit retinal damage. The invention also includes methods for treating and prophylaxis against decreased blood flow or nutrient supply to retinal tissue or optic nerve, or treatment or prophylaxis against retinal trauma or optic nerve injury. Subjects for treatment according to such therapeutic methods of the invention may be suffering or susceptible to retinal ischemia that is associated with atherosclerosis, venous capillary insufficiency, obstructive arterial or venous retinopathies, senile macular degeneration, cystoid macular edema or glaucoma, or the retinal ischemia may be associated with a tumor or injury to the mammal. Intravitreal injection of a compound of the invention also may be a preferred administration route to provide more direct treatment to the ischemic retina.

The invention also provides methods for treatment of a subject suffering from shingles as well as treatment of a person suffering from or susceptible to migraines, particularly to alleviate the pain and discomfort associated with those disorders. As discussed above, compounds of the invention are also useful to treat persons suffering from various types of pain, including chromic pain. These methods comprise administration of an effective amount of one or more compounds of the invention to a patient in need of treatment.

The invention further provides a method of treating Korsakoffs disease, a chronic alcoholism-induced condition, comprising administering to a subject including a mammal, particularly a human, one or more compounds of the invention in an amount effective to treat the disease. Compounds of the invention are anticipated to have utility for the attenuation of cell loss, hemorrhages and/or amino acid changes associated with Korsakoff's disease.

As discussed above, the invention also includes methods for treating a person suffering from or susceptible to cerebral palsy, emesis, narcotic withdrawal symptoms and age-dependent dementia, comprising administering to a subject including a mammal, particularly a human, one or more compounds of the invention in an amount effective to treat the condition.

The invention also includes methods for treatment of infections, including Gram-negative and Gram-positive bacterial infections, comprising administering a combination of 1) an aminoglycoside antibiotic, and 2) a compound of the invention.

A wide variety of aminoglycoside antibiotics are suitable for use in the formulations of the invention. Typically, suitable aminoglycoside antibiotics contain two or more amino sugars (aminoglycosides) connected to an amino-cyclitol nucleus. Exemplary aminoglycoside antibiotics preferred for use in formulations of the present invention include clinical agents such as gentamycin, amikacin, kanamycin, streptomycin, paromoycin, neomycin, netilmicin and tobramycin. Other suitable aminoglycosides include seldomycins, sisomycins, aurimycin, lividomycins, streptothricins, hybrimycins, coralinomycin, butirosin, strepomutins, nebramycins, tenebrimycins, ribostamycins, destomycins, trehalosamines, myomycins, fortimicins, mutamicins and kasugamycin. Suitable aminoglycoside antibiotics are also disclosed in U.S. Patents Nos. 5,508,269; 4,645,760; and 4,380,625. It should be appreciated however that the present invention is not limited by any particular aminoglycoside antibiotic, and the invention is applicable to any aminoglycoside antibiotic now known or subsequently discovered or developed. The aminoglycoside and one or more compounds of the invention may be administered simultaneously, in the same or different pharmaceutical formulations, or sequentially. Preferably, the components of the combination are administered substantially simultaneously, e.g. in a unitary pharmaceutical composition containing the two components. Preferred methods and compositions that comprise an aminoglycoside in combination with a compound of the invention will be effective against infections previously treated with aminoglycoside antibiotics, but with the significant advantage of decreased occurrence of ototoxicity relative to use of an aminoglycoside antibiotic alone.

As discussed above, preferred compounds of the invention in a standard anticonvulsant *in vivo* audiogenic test, such as the audiogenic mouse assay of Example 11 which follows, where DBA/2 mice about 20-23 days old are injected intraperitoneally with a test compound 30 minutes prior to being placed in a bell jar with exposure to auditory stimulus of 12KHz sine wave at 110-120 db. References herein *in vivo* "audiogenic assay" are intended to refer to that protocol. Generally preferred compounds exhibit 20% or more inhibition (relative to subjects treated with vehicle control only) at a dose of 20 mg/kg, more preferably about 50% or more inhibition at a dose of 20 mg/kg in such an *in vivo* audiogenic assay. As discussed above, activity in the audiogenic assay has been recognized as indicative that a test compound has neuroprotective properties. See, e.g., M. Tricklebank et al., European Journal of Pharmacology*, supra*; T. Seyfried, Federation Proceedings*, supra.*

The invention also provides methods for determining binding activity of compounds of the invention as well as in vitro and *in vivo* binding activity diagnostic methods using one or more radiolabelled compounds of the invention, e.g., a compound of the invention that is labeled with ¹²⁵I, tritium, ³²P, ⁹⁹Tc, or the like, preferably ¹²⁵I. For instance, a compound of the invention having a phenyl or other aryl substituent that is ring substituted with one or more ¹²⁵I groups can be administered to a mammal and the subject then scanned for binding of the compound. Specifically, single photon emission computed tomography ("SPECT") can be employed to detect such binding. Such an analysis of the mammal could e.g. aid in the diagnosis and treatment of acute cerebral ischemia. That is, a labeled compound of the invention will selectively bind to ischemic tissue of e.g. a subject's brain to differentiate between ischemic and non-ischemic tissue and thereby assess trauma or other injury to the brain.

Accordingly, the invention includes compounds of the invention that contain a radiolabel such as ¹²⁵I, tritium, ³²P, ⁹⁹Tc, or the like, preferably ¹²⁵I. Such radiolabelled compounds can be suitably prepared by procedures known in the synthesis art. For example, a compound of the invention having an aromatic group, such as phenyl, that has a bromo or chloro ring substituent can be employed in an exchange labeling reaction to provide the corresponding compound having an ¹²⁵I ring substituent.

Compounds of the invention may be used in therapy in conjunction with other medicaments. For example, for treatment of a stroke victim or a person susceptible to stroke, one or more compounds of Formula I may be suitably administered together with a pharmaceutical targeted for interaction in the blood clotting mechanism such as streptokinase, tPA, urokinase and other agents that lyse clots. Also, one or more compounds of the invention may be administered together with agents such as heparin and related heparin-based compounds, acenocoumarol or other known anticoagulants.

The compounds of this invention can be administered intranasally, orally or by injection, e.g., intramuscular, intraperitoneal, subcutaneous or intravenous injection, or by transdermal, intraocular or enteral means. The optimal dose can be determined by conventional means. Compounds of the invention are suitably administered to a subject in the protonated and water-soluble form, e.g., as a pharmaceutically acceptable salt of an organic or inorganic acid, e.g., hydrochloride, sulfate, hemi-sulfate, phosphate, nitrate, acetate, oxalate, citrate, maleate, mesylate, etc.

Compounds of the invention can be employed, either alone or in combination with one or more other therapeutic agents as discussed above, as a pharmaceutical composition in mixture with conventional excipient, i.e., pharmaceutically acceptable organic or inorganic carrier substances suitable for parenteral, enteral or intranasal application which do not deleteriously react with the active compounds and are not deleterious to the recipient thereof. Suitable pharmaceutically acceptable carriers include but are not limited to water, salt solutions, alcohol, vegetable oils, polyethylene glycols, gelatin, lactose, amylose, magnesium stearate, talc, silicic acid, viscous paraffin, perfume oil, fatty acid monoglycerides and diglycerides, petroethral fatty acid esters, hydroxymethyl-cellulose, polyvinylpyrrolidone, etc. The pharmaceutical preparations can be sterilized and if desired mixed with auxiliary agents, e.g., lubricants, preservatives, stabilizers, wetting agents, emulsifiers, salts for influencing osmotic pressure, buffers, colorings, flavorings and/ or aromatic substances and the like which do not deleteriously react with the active compounds.

For parenteral application, particularly suitable are solutions, preferably oily or aqueous solutions as well as suspensions, emulsions, or implants, including suppositories. Ampules are convenient unit dosages.

For enteral application, particularly suitable are tablets, dragees or capsules having talc and/or carbohydrate carrier binder or the like, the carrier preferably being lactose and/or corn starch and/or potato starch. A syrup, elixir or the like can be used wherein a sweetened vehicle is employed. Sustained release compositions can be formulated including those wherein the active component is protected with differentially degradable coatings, e.g., by microencapsulation, multiple coatings, etc.

For topical applications, formulations may be prepared in a topical ointment or cream containing one or more compounds of the invention. When formulated as an ointment, one or more compounds of the invention suitably may be employed with either a paraffinic or a water-miscible base. The one or more compounds also may be formulated with an oil-in-water cream base. Other suitable topical formulations include e.g. lozenges and dermal patches.

Intravenous or parenteral administration, e.g., sub-cutaneous, intraperitoneal or intramuscular administration are preferred. The compounds of this invention are particularly valuable in the treatment of mammalian subjects, e.g., humans, to provide neuroprotective therapy and/or prophylaxis. Typically, such subjects include those afflicted with neurodegenerative diseases such as Parkinson's disease, Huntington's disease, Amyotrophic Lateral Sclerosis, Alzheimer's disease, Down's Syndrome and Korsakoffs disease. Also suitable for treatment are those subjects suffering from or likely to suffer from nervous system dysfunctions resulting from, for example, epilepsy or nerve cell degeneration which is the result of hypoxia, hypoglycemia, brain or spinal chord ischemia or brain or spinal chord trauma. As discussed above, typical candidates for treatment include heart attack, stroke, brain or spinal cord injury patients, patients undergoing major surgery where brain or spinal cord ischemia is a potential complication and patients such as divers suffering from decompression sickness due to gas emboli in the blood stream.

It will be appreciated that the actual preferred amounts of active compounds used in a given therapy will vary according to the specific compound being utilized, the particular compositions formulated, the mode of application, the particular site of administration, etc. Optimal administration rates for a given protocol of administration can be readily ascertained by those skilled in the art using conventional dosage determination tests conducted with regard to the foregoing guidelines. In general, a suitable effective dose of one or more compounds of the invention, particularly when using the more potent compound(s) of the invention, will be in the range of from 0.01 to 100 milligrams per kilogram of bodyweight of recipient per day, preferably in the range of from 0.01 to 20 milligrams per kilogram bodyweight of recipient per day, more preferably in the range of 0.05 to 4 milligrams per kilogram bodyweight of recipient per day. The desired dose is suitably administered once daily, or several sub-doses, e.g. 2 to 4 sub-doses, are administered at appropriate intervals through the day, or other appropriate schedule. Such sub-doses may be administered as unit dosage forms, e.g., containing from 0.05 to 10 milligrams of compound(s) of the invention, per unit dosage, preferably from 0.2 to 2 milligrams per unit dosage.

Compounds of the invention also should be useful as rubber accelerators. See U.S. Patent No. 1,411,713 for a discussion of rubber accelerator applications.

The following non-limiting examples are illustrative of the invention, wherein Example 2 is a reference example not comprised by the invention.

### GENERAL COMMENTS

Melting points were determined in open capillary tubes on a Mel-Temp II apparatus and are uncorrected. Yields are of isolated products and were not optimized. ¹H-NMR were run on a Varian Gemini 300 MHz spectrophotometer and the chemical shifts were reported in ppm (δ) relative to the residual signal of deutrated solvent (CHD₂OD 3.30, CDCl₃ 7.26). HPLC purity determinations were carried out using a Beckman 126 gradient system with UV detection at 220 nm. Linear 30 minutes gradient:2 to 98% CH₃CN in H₂O (0.1% TFA) Column: Ultrasphere ODS (AC-2) 5mm 4.6 X 250 mm with C-18 guard column, flow rate 1 ml/min.

### EXAMPLE 1: Synthesis of N-(4-methylbenzoyl)-N'-(4-isopropylphenyl)guanidine, hydrochloride (Formula I: hydrochloride salt of R=4-CH₃C₆H₄; each R¹=R²=H; X=chemical bond; R³=4-isopropylphenyl)

### Part I: 4-methylbenzoylcyanamide

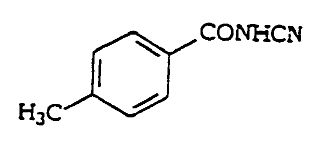

To a solution of cyanamide (1.05 g, 0.025 mmol) in 25 ml of sodium hydroxide (10%) was added slowly to a solution of 4-methylbenzoyl chloride (3.3 ml, 0.025 mmol) in ether (8 ml). The reaction mixture was stirred at room temperature for 1 hour. The reaction mixture then was cooled in ice bath and acidified with hydrochloric acid (10%) to pH 2. The white solid separated was filtered, washed with water, later hexanes and dried under high vacuum to give the N-(4-methylbenzoyl)cyanamide (3.4 g); m.p. 140-142°C (lit m.p. 149-150°C); purity 88% HPLC; ¹H-NMR (CD₃OD) δ 2.42 (s, 3H, CH₃), 7.38 (d, 2H, ArH), 7.78 (d, 2H, ArH).

### Part II: N-(4-methylbenzoyl)-N'-(4-isopropylphenyl)guanidine, hydrochloride

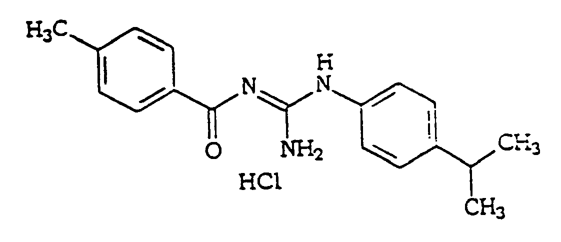

A mixture of N-(4-methylbenzoylcyanamide) (160 mg, 1 mmol) and 4-isopropylaniline•hydrochloride (185 mg [prepared from 4-isopropylaniline and hydrogen chloride (1M in ether)]) in toluene (4 ml) was refluxed for 3 hours. The reaction mixture was cooled to room temperature, the precipitated white solid was filtered, washed with toluene and finally with hexanes to afford the title product, (268 mg, 78%); m.p. 208-210°C; purity 99.3% (HPLC); ¹H-NMR (CD₃OD) δ 1.28 (2s, 6H, CH₃), 2.42 (s, 3H, Ar-CH₃), 3.02 (m, 1H, CH), 7.38 (d, 2H, ArH), 7.42 (m, 4H, ArH), 8.0 (d, 2H, ArH).

### EXAMPLE 2: Preparation of N-(1-adamantancarbonyl)-1-indolinylcarboxamidine, hydrochloride ((Formula II: hydrochloride salt of R=1-adamantyl; each R¹ =R²=R³=H)

### Part I: Adamantancarbonylcyanamide

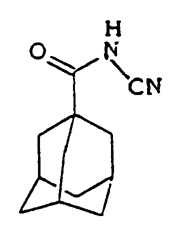

To a solution of cyanamide (2.52 g, 0.06 mol) in 24 ml of sodium hydroxide (10%) was added slowly to a solution of 1-adamantanecarbonylchloride (4g, 0.02 mol) in ether (15 ml). The reaction mixture was stirred at room temperature overnight. The reaction mixture was extracted with ether and the aqueous layer was cooled in ice bath and acidified with hydrochloric acid (10%) to pH 2. The precipitated white solid was filtered, washed with water, later hexanes and dried under high vacuum to give the N-(1-adamantancarbonyl)cyanamide (3.2 g, 78%); m.p. 164-166°C (lit m.p. 168-170°C); purity 94% HPLC; ¹H-NMR (CD₃OD) δ 1.45-2.16 (m, 15H, CH₂ and CH).

### Part II: N-(1-adamantancarbonyl)-1-indolinylcarboxamidine, hydrochloride

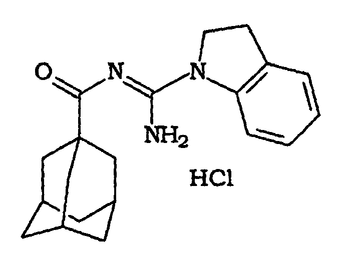

A mixture of N-(1-adamantanecarbonyl)cyanamide (200 mg, 0.88 mmol) and indoline hydrochloride (155 mg [prepared from indoline and hydrogen chloride (1M in ether)]) in toluene (4 ml) was refluxed for 3 hours. The reaction mixture was cooled to room temperature, the precipitated white solid was filtered, washed with toluene and finally with hexanes to afford the title product, (339 mg, 78%); m.p. 252-256°C; Purity 99% (HPLC); ¹H-NMR (CD₃OD) δ 1.65 (m, 15H, CH₂ and CH), 3.2 (t, 2H, ArCH₂), 4.2 (t, 2H, NCH₂), 7.25 (m, 1H, arH), 7.36 (m, 2H, ArH), 7.42 (m, 1H, ArH).

### EXAMPLE 3: Preparation of N-(phenylacetyl)-N'-(4-t-butylphenyl)guanidine, hydrochloride (Formula I: hydrochloride salt of R=C₆H₅; each R¹=R²=H; X=chemical bond; R³=4-tert-butylphenyl)

### Part I: Phenylacetylcyanamide

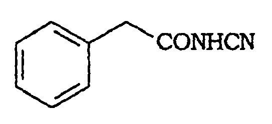

This compound was prepared in 83% yield by the method described in Example 2, Part I above using phenylacetyl chloride in place of adamantane-1-carbonyl chloride. Phenylacetylcyanamide: a white solid; purity 92% (HPLC); ¹H-NMR (CD₃OD) δ 2.3 (s, 2H), 7.25-7.45 (m, 5H).

### Part II: N-(Phenylacetyl)-N'-(4-t-butylphenyl)guanidine, hydrochloride

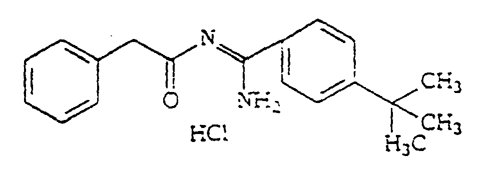

Synthesis of this compound was achieved by the method set forth in Example 1, Part II above with the use of phenylacetylcyanamide in place of 4-methylbenzoylcyanamide and using 4-t-butylaniline hydrochloride instead of 4-isopropylaniline hydrochloride respectively. Yield 69%; m.p. 182-186°C; Purity 86% (HPLC); ¹H-NMR (CD₃OD) δ 1.38 (s, 9H, CH₃), 3.84 (s, 2H, CH₂), 7.3 (d, 2H, ArH), 7.3-7.45 (m, 5H, ArH), 7.6 (d, 2H, ArH).

### EXAMPLE 4: Preparation of N-(2-thiophenecarbonyl)-N'-(4-benzyloxyphenyl)guanidine, hydrochloride (Formula I: hydrochloride salt of R=2-thiophenyl; each R¹=R²=H; X=chemical bond; R³=4-C₆H₅CH₂OC₆H₄-)

### Part I: (2-Thiophenecarbonyl)cyanamide

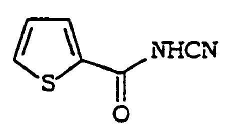

This compound was prepared in 73% yield by the method described in Example 2, Part I above using 2-thiophenecarbonylchloride in place of adamantane-1-carbonyl chloride. N-(2-thiophenecarbonyl)cyanamide: a white solid; purity 96% (HPLC); ¹H-NMR (CD₃OD) δ 7.2 (m, 1H), 7.8 (d, 1H), 7.9 (d, 1H).

### Part II: N-(2-Thiophenecarbonyl)-N'-(4-benzyloxyphenyl)guanidine, hydrochloride

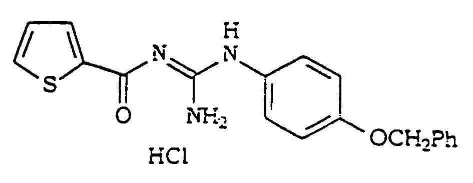

Synthesis of this compound was achieved by the method set forth in Example 1, Part II above with the use of N-(2-thiophenecarbonyl)cyanamide in place of N-(4-methylbenzoylcyanamide) and using 4-benzylcyanamide and using 4-benzyloxyaniline hydrochloride instead of 4-isopropylaniline hydrochloride respectively. Yield 61%; m.p. 198-202°C; Purity 93% (HPLC); ¹H-NMR (CD₃OD) δ 5.18 (s, 2H, CH₂), 7.18 (d, 2H), 7.22-7.4 (m, 6H), 7.44 (d, 2H), 7.9 (d, 1H), 8.18 (d, 1H).

### EXAMPLE 5 : Preparation of N-(4-methylbenzoyl)-N'-(4-phenylbutyl)guanidine, hydrochloride (Formula I: hydrochloride salt of R=4-CH₃C₆H₄; each R¹=R²=H; X=CH₂CH₂CH₂CH₂; R³=C₆H₅)

### Part I: N-(4-Methylbenzoyl)-S-methylisothiourea

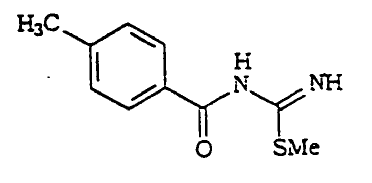

A solution of 2-methyl-2-thiopseudourea sulfate (6.9 g, 0.025 mol) in 30 ml of sodium hydroxide (4%) was added a solution of 4-methylbenzoyl chloride (3.4 g, 0.022 mol) in ether (10 ml) at room temperature. The reaction mixture was stirred overnight and the precipitated solid was filtered, washed with water, later hexanes and dried under high vacuum. N-(4-methylbenzoyl)-S-methylisothiourea: yield 4.60 g (quantitative); purity 98% (HPLC); ¹H-NMR (CD₃OD) δ 2.4 (s, 3H, CH₃), 2.6 (s, 3H, SMe), 7.2 (d, 2H, ArH), 8.1 (d, 2H, ArH).

### Part II: N-(4-methylbenzoyl)-N'-(4-phenylbutyl)guanidine, hydrochloride

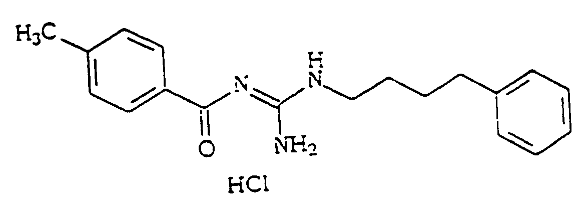

Phenylbutyl amine (0.75 ml, 4.75 mmol) and triethylamine (0.7 ml, 5 mmol) was added to a suspension of the thiourea derivative (1.04 g, 5 mmol), prepared in Part I, in touene (10 ml). The reaction mixture was heated in an oilbath to reflux and maintained at reflux for 3 hours. The free base separated on cooling was filtered, washed with hexanes and dried to afford the solid (1.3 g).

The free base (1.3 g) was dissolved in methanol (30 ml) and dichloromethane (25 ml) and cooled in an ice water bath. Hydrogen chloride (1M in ether, 20 ml) was added, stirred for 30 minutes, concentrated under reduced pressure. N-(4-methylbenzoyl)-N'-(4-phenylbutyl)guanidine, hydrochloride: white solid (1.43 g, 84%); m.p. 166-170°C; Purity: 99% (HPLC); ¹H-NMR (CD₃OD) δ 1.74 (m, 4H, CH₂), 2.43 (s, 3H, CH₃), 2.69 (t, 2H, CH₂), 3.38 (t, 2H, CH₂), 7.2 (m, 5H, ArH), 7.4 (d, 2H, ArH), 7.9 (d, 2H, ArH).

### EXAMPLE 6: Preparation of N-(4-methoxybenzoyl)-N'-(4-phenylbutyl)guanidine, hydrochloride (Formula I: hydrochloride salt of R=4-CH₃OC₆H₄; each R¹=R²=H; X=CH₂CH₂CH₂CH₂; R³=C₆H₅)

### Part I: N-(4-Methoxybenzoyl)-S-methylisothiourea

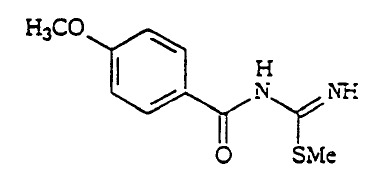

Synthesis of this compound was achieved by the method set forth in Example 5, Part I above with the use of 4-methoxybenzoyl chloride in place of 4-methylbenzoyl chloride. N-(4-methoxybenzoyl)-S-methylisothiourea: white solid (83% yield); Purity 99% (HPLC); ¹H-NMR (CD₃OD) δ 2.57 (s, 3H, SCH₃), 3.86 (s, 3H, OCH₃), 6.94 (d, 2H, ArH), 8.15 (d, 2H, ArH).

### Part II: N-(4-Methoxybenzoyl)-N'-(4-phenylbutyl)guanidine, hydrochloride

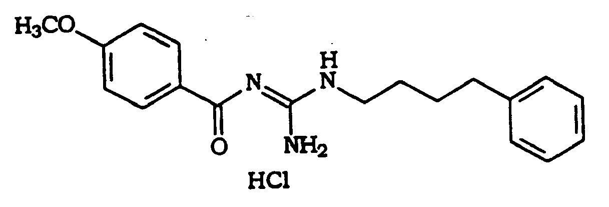

Preparation of this compound was carried out by the method as described in Example 5, Part II above using 4-methoxybenzoyl-S-methylisothiourea in place of 4-methylbenzoyl-S-methylisothiourea. N-(4-methoxybenzoyl)-N'-(4-phenylbutyl)guanidine, hydrochloride: white solid (55%); m.p. 172-174°C; Purity 99% (HPLC); ¹H-NMR (CD₃OD) δ 1.74 (m, 4H, CH₂), 2.67 (t, 2H, CH₂), 3.37 (t, 2H, CH₂), 3.89 (s, 3H, OCH₃), 7.10 (d, 2H, ArH), 7.22 (m, 5H, ArH), 7.97 (d, 2H, ArH).

### EXAMPLE 7: N-(2-thiophenecarbonyl)-N'-(2-phenylethyl)guanidine, hydrochloride (Formula I: hydrochloride salt of R=2-thiophenyl; each R¹=R²=H; X=CH₂CH₂; R³=C₆H₅)

### Part I: N-(2-Thiophenecarbonyl)-S-methylisothiourea

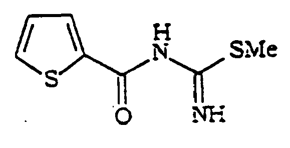

Synthesis of this compound was achieved by the method set forth in Example 5, Part I above with the use of 2-thiophenecarbonyl chloride in place of 4-methylbenzoyl chloride. N-(2-thiophenecarbonyl)-S-methylisothiourea: white solid (73% yield); Purity 91.2% (HPLC); ¹H-NMR (CD₃QD) δ 2.76 (s, 3H, SCH₃), 7.26 (m, 1H, ArH), 8.01 (d, 1H, ArH), 8.12 (d, 1H, ArH).

### Part II: N-(2-Thiophenecarbonyl)-N'-(2-phenylethyl)guanidine, hydrochloride

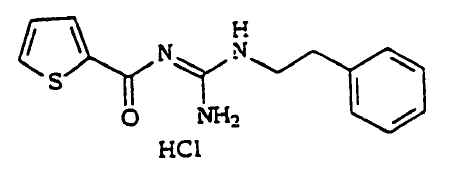

Preparation of this compound was carried out by the method as described in Example 5, Part II above using N-(2-thiophenecarbonyl)-S-methylisothiourea in place of 4-methylbenzoyl-S-methylisothiourea and 2-phenylethylamine instead of 4-phenylbutylamine respectively. N-(2-thiophenecarbonyl)-N'-(2-phenylethyl)guanidine, hydrochloride: white solid (58%); m.p. 198-200°C; Purity 97% (HPLC); ¹H-NMR (CD₃OD) δ 3.00 (t, 2H, CH₂), 3.65 (t, 2H, CH₂), 7.2 (d, 1H, ArH), 7.28 (m, 5H, ArH), 7.97 (d, 2H, ArH).

### EXAMPLE 8: N-(4-Butoxybenzoyl)-N'-[2-(indol-3-yl)ethyl]guanidine, hydrochloride (Formula I: hydrochloride salt of R=4-CH₃(CH₂)₃OC₆H₄; each R¹=R²=H; X=CH₂CH₂; R³=3-indoly)

### Part I: N-(4-Butoxybenzoyl)-S-methylisothiourea

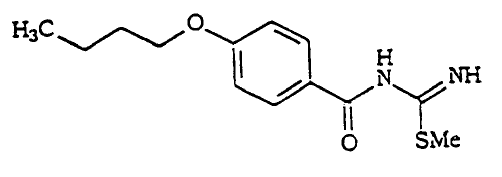

This compound was prepared following the method as described in Example 6, Part I above using 4-butoxybenzoyl chloride in place of 4-methoxybenzoyl chloride. N-(4-butoxybenzoyl)-S-methylisothiourea: white solid (85%); Purity 95% (HPLC); ¹H-NMR (CD₃OD) δ 1.0 (t, 3H, CH₃), 1.50 (m, 2H, CH₂), 1.78 (m, 2H, CH₂), 2.56 (s, 3H, SCH₃), 4.03 (t, 2H, OCH₂), 6.93 (d, 2H, ArH), 8.14 (d, 2H, ArH).

### Part II: N-(4-Butoxybenzoyl)-N'-[2-(indol-3-yl)ethyl]guanidine, hydrochloride

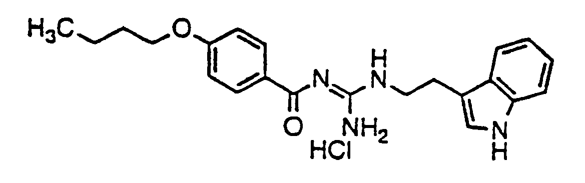

Preparation of this compound was carried out by the method as described in Example 5, Part II above using N-(4-butoxybenzoyl)-S-methylisothiourea in place of 4-methylbenzoyl-S-methylisothiourea and 3-(2-aminoethyl)indole (tryptamine) instead of 4-phenylbutylamine respectively. N-(4-butoxybenzoyl)-N'-[2-(indol-3-yl)ethyl]guanidine, hydrochloride: solid (yield 28%); m.p. 158-162°C; Purity 95% (HPLC); ¹H-NMR (CD₃OD) δ 0.99 (t, 3H, CH₃), 1.49 (m, 2H, CH₂), 1.76 (m, 2H, CH₂), 3.28 (t, 2H, CH₂), 3.69 (t, 2H, CH₂), 4.05 (t, 2H, CH₂), 7.04 (m, 4H, ArH), 7.2 (d, 1H), 7.32 (d, 1H, ArH), 7.56 (d, 1H, ArH), 7.86 (d, 2H, ArH).

### EXAMPLE 9: N-(4-Methylbenzoyl)-N'-(4-phenylbutyl)-N"-methylguanidine, hydrochloride (Formula I: hydrochloride salt of R=4-CH₃C₆H₄; first R¹=CH₃, second R¹=H; R²=H; X=CH₂CH₂CH₂CH₂; R³=C₆H₅)

### Part I: Dimethyl N-(4-methylbenzoylcarbimidodithiolate)

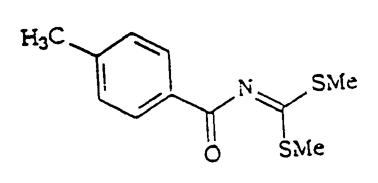

A mixture of 4-methylbenzamide (1.35 g, 0.01 mol) in anhydrous tetrahydrofuran (50 ml), carbon disulfide (3 g, 0.039 mol), and methyl iodide (4.5 g, 0.032 mol), and sodium hydride (0.85 g, 60% dispersion in oil, 0.02 mol) was stirred at room temperature overnight. The reaction mixture was poured onto ice, extracted with ethyl acetate (3 X 30 ml), washed with water, dried and concentrated to give an oil. This solidified on standing and was crystallized from hexanes as bright yellow crystals (0.8 g); m.p. 57-59°C (lit 60-61°C); ¹H-NMR (CDCl₃) δ 2.40 (s, 3H, Ar-Me), 2.57 (s, 6H, SMe), 7.25 (d, 2H, ArH), 7.98 (d, 2H, ArH).

### Part II: N-(4-methylbenzoyl)-N'-(4-phenylbutyl)-S-Methylthiourea

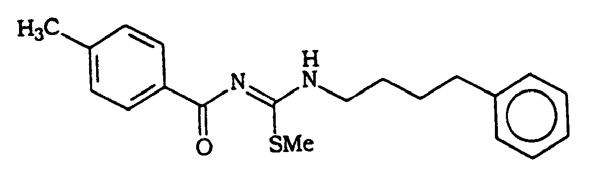

A mixture of dimethyldithiolate (240 mg, mmol, prepared as in Part I) and phenybutylamine (150 mg, mmol) in ethanol (5 ml) was stirred overnight at room temperature. The reaction mixture was concentrated and the oil obtained was repeatedly coevaporated with dichloromethane, upon which solid separated. This solid was triturated with hexanes, filtered and dried. N-(4-methylbenzoyl)-N'-(4-phenylbutyl)-S-Methylthiourea: white solid (80 mg); ¹H-NMR (CDCl₃) δ 1.85 (m, 4H, CH₂), 2.38 (s, 3H, ArMe), 2.61 (s and t, 5H, CH₂ and Sme), 3.35 (t, 2H, CH₂), 7.2 (m, 5H, ArH), 7.28 (d, 2H, ArH), 8.12 (d, 2H, ArH).

### Part III: N-(4-Methylbenzoyl)-N'-(4-phenylbutyl)-N"-methylguanidine, hydrochloride

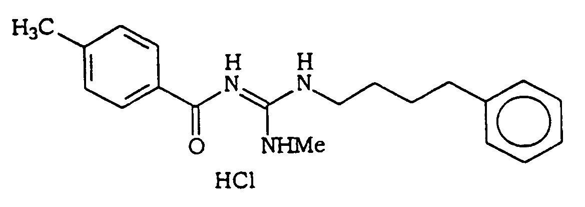

A solution of N-(4-methylbenzoyl)-N'-(4-phenylbutyl)-S-Methylthiourea (80 mg, prepared as in Part II) in 5 ml of methylamine (2M in methanol) was stirred at room temperature for 48 hours. After removal of the solvent the residue was dissolved in methanol (3 ml), and a ethereal solution of hydrogen chloride (5 ml) was added. The solid separated was filtered, washed with ether and dried. Pale yellow solid (50 mg); Purity: 93% (HPLC); ¹H-NMR (CD₃OD) δ 1.71 (m, 4H, CH₂), 2.40 (s, 3H, ArCH₃), 2.65 (t, 2H, CH₂), 3.05 (s, 3H, NMe), 3.4 (t, 2H, CH₂), 7.13 (m, 5H, Ar), 7.35 (d, 2H, ArH), 7.82 (d, 2H, ArH).

### EXAMPLE 10: N-(2,6-Dichlorophenylacetyl)-N'-benzylguanidine hydrochloride (Formula I': hydrochloride salt of R=2,6-di-C₆H₃; both R¹=H; R²=H; X=CH₂; R³=C₆H₅)

### Part I: Benzylguanidine hydrochloride

A mixture of benzylamine hydrochloride (4.3g, 0.03 mol) and cyanamide (1.3g, 0.031 mol) in xylenes (15 ml) was heated to reflux for 6 hours. After concentration, the reaction mixture was triturated with ether and the solid separated was filtered and crystallized from methanol to provide a colorless solid (2.38 g); purity: 96.8% (HPLC); 1H-NMR (CD30D) δ 4.41 (s, 2H, CH₂), 7.32-7.37 (m, 5H, Ar).

### Part II: N-(2,6-Dichlorophenylacetyl)-N'-benzylguanidine hydrochloride

To sodium ethoxide [prepared by reacting sodium (60 mg, 2.61 mmol) and anhydrous ethanol (5 ml)] benzylguanidine hydrochloride (580 mg, 3.12 mmol) was added and refluxed in an oilbath for 1 hour. The reaction mixture was cooled to room temperature and insoluble materials filtered. Methyl 2,6-dichlorophenylacetate (285 mg, 1.3 mmol) (CH₃(C=O)CH₂(2,6,-di-ClC₆H₃) was added to the filtrate and refluxed for 2 hours. After cooling to room temperature the reaction mixture was concentrated and converted to the hydrochloride salt by the addition of hydrogen chloride (1M in ether) to provide 310 mg of N-(2,6-dichlorophenylacetyl)-N'-benzylguanidine hydrochloride as a white solid, purity 89.3% (HPLC); ¹H-NMR (CD₃OD) δ 4.38 (s, 4H, CH₂), 7.32-7.41 (m, 8H, Ar).

### EXAMPLE 11 In vivo Anticonvulsant activity in the DBA/2 mouse model (Mouse audiogenic assay)

The in vivo potency of compounds of the invention is exemplified by data summarized in the Table I below and obtained pursuant to the following protocol.

Compounds were tested for their effectiveness in preventing seizures in DBA/2 mice which have a unique sensitivity to auditory stimulation. Exposure to loud high-frequency sounds can trigger seizure activity in these animals. This sensitivity develops from postnatal day 12 and peaks around day 21 and slowly diminishes as the animals mature. The unusual response to auditory stimulation in this strain of mouse is believed to be due to a combination of early myelination (causing an unusually low excitatory threshold) and delayed development of inhibitory mechanisms.

Mice were injected intraperitoneally with the compound specified in Table I below or with vehicle control, 30 minutes prior to being placed in a bell jar and turning on the auditory stimulus (12 KHz sine wave at 110-120 db). Administered doses are specified in Table I as milligram of compound per kilogram bodyweight of mouse. The auditory stimulus was left on for 60 seconds and mice reactions were timed and recorded. Percentage inhibition was determined with reference to vehicle controls. Results are shown in the Table I below. All compounds were tested in HCl salt form.

**Table I**

| Compound Name | Audiogenic Response | |
|---|---|---|
| | Dose | % Inhib. |
| | (mg/kg) | |
| N-(4-methylbenzoyl)-N'-(4-isopropylphenyl)guanidine | 20 | 30 |
| N-(4-methylbenzoyl)-N'-(4-phenylbutyl)guanidine | 20 | 75 |
| | 10 | 16 |
| N-(4-methoxybenzoyl)-N'-(4-phenylbutyl)guanidine | 20 | 42 |
| N-(4-methoxyphenyl)-N'-(4-isopropylphenyl)guanidine | 20 | 56 |
| N-(4-ethoxyphenyl)-N'-(4-phenylbutyl)guanidine | 20 | 60 |
| N-(4-butoxyphenyl)-N'-(4-phenylbutyl)guanidine | 20 | 97 |

## Claims

1. A compound selected from:
N-(4-methylbenzoyl)- N'-methyl-N'-(3-methylthiophenyl)guanidine;
N-(4-methylbenzoyl)-N'-methyl-N'-(3-iodophenyl)guanidine;
N-(4-methylbenzoyl)-N'-(1-naphthyl)guanidine;
N-(4-methylbenzoyl)-N'-(4-benzyloxyphenyl)guanidine;
N-(4-methylbenzoyl)-N'-(4-tertbutylphenyl)guanidine;
N-(4-methylbenzoyl)-N'-(4-isopropylphenyl)guanidine;
N-(4-methylbenzoyl)-N'-(2,5-dibromophenyl)guanidine;
N-(4-methylbenzoyl)-N'-(4-isopropoxyphenyl)guanidine;
N-(4-methylbenzoyl)-N'-(3,4,5-trimethoxyphenyl)guanidine;
N-(4-methylbenzoyl)-N'-(2-isopropylphenyl)guanidine;
N-(2,5-dichlorobenzoyl)-N'-methyl-N'-(3-iodophenyl)guanidine;
N-(2,5-dichlorobenzoyl)- N'-methyl-N'-(3-methytthiophenyl)guanidine;
N-(2,5-dichlorobenzoyl)-N'-(1-naphthyl)guanidine;
N-(2,5-dichlorobenzoyl)-N'-(4-benzyloxyphenyl)guanidine;
N-(2, 5-dichlorobenzoyl)-N'-(4-isopropylphenyl)guanidine;
N-(2,5-dichlorobenzoyl)-N'-(4-tertbutylphenyl)guanidine;
N-(2,5-dichlorobenzoyl)- N'-methyl-N'-(4-isopropylphenyl)guanidine;
N-(phenylacetyl)-N'-(4-benzyloxyphenyl)guanidine;
N-(phenylacetyl)-N'-(4-isopropylphenyl)guanidine;
N-(phenylacetyl)-N'-(4-tert-butylphenyl)guanidine;
N-(phenylacetyl)-N'-(4-isopropoxyphenyl)guanidine;
N-(phenylacetyl)-N'-(4-isopropylphenyl)-N'-methylguanidine;
N-(adamantan-1-carbonyl)-N'-methyl-N'-(3-iodophenyl)guanidine;
N-(adamantan-1-carbonyl)-N'-(1-naphthyl)guanidine;
N-(adamantan-1-carbonyl)-N'-(4-benzyloxyphenyl)guanidine;
N-(adamantan-1-carbonyl)-N'-(4-isopropylphenyl)guanidine;
N-(adamantan-1-carbonyl)-N'-(4-tert-butylphenyl)guanidine;
N-(adamantan-1-carbonyl)-N'-(2,5-dibromophenyl)guanidine;
N-(adamantan-1-carbonyl)-N'-(4-isopropylphenyl)-N'-methyl)guanidine;
N-(4-chlorobenzoyl)-N'-methyl-N'-(3-iodophenyl)guanidine;
N-(4-chlorobenzoyl)-N'-methyl-N'-(3-methylthiophenyl)guanidine;
N-(4-chlorobenzoyl)-N'-(1-naphthyl)guanidine;
N-(4-chlorobenzoyl)-N'-(4-benzyloxyphenyl)guanidine;
N-(4-chlorobenzoyl)-N'-(4-isopropylphenyl)guanidine;
N-(4-chlorobenzoyl)-N'-(4-tert-butylphenyl)guanidine;
N-(4-chlorobenzoyl)-N'-(2,5-dibromophenyl)guanidine;
N-(3,4-dichlorobenzoyl)-N'-methyl-N'-(3-iodophenyl)guanidine;
N-(3,4-dichlorobenzoyl)-N'-(1-naphthyl)guanidine;
N-(3,4-dichlorobenzoyl)-N'-(4-benzyloxyphenyl)guanidine;
N-(3,4-dichlorobenzoyl)-N'-(4-isopropylphenyl)guanidine;
N-(3,4-dichlorobenzoyl)-N'-(4-tert-butylphenyl)guanidine;
N-(3,4-dichlorobenzoyl)-N'-methyl-N'-(4-isopropylphenyl)guanidine;
N-(thiophen-2-carbonyl)-N'-methyl-N'-(3-iodophenyl)guanidine;
N-(thiophen-2-carbonyl)-N'-methyl-N'-(3-methylthiophenyl)guanidine;
N-(thiophen-2-carbonyl)-N'-(1-naphthyl)guanidine;
N-(thiophen-2-carbonyl)-N'-(4-benzyloxyphenyl)guanidine;
N-(thiophen-2-carbonyl)-N'-(4-isopropylphenyl)guanidine;
N-(thiophen-2-carbonyl)-N'-(4-tert-butylphenyl)guanidine;
N-(thiophen-2-carbonyl)-N'-methyl-N'-(4-isopropylphenyl)guanidine;
N-(furan-2-carbonyl)-N'-methyl-N'-(3-iodophenyl)guanidine;
N-(furan-2-carbonyl)-N'-methyl-N'-(3-methylthiophenyl)guanidine;
N-(furan-2-carbonyl)-N'-(1-naphthyl)guanidine;
N-(furan-2-carbonyl)-N'-(4-benzyloxyphenyl)guanidine;
N-(furan-2-carbonyl)-N'-(4-isopropylphenyl)guanidine;
N-(furan-2-carbonyl)-N'-(4-tert-butylphenyl)guanidine;
N-(furan-2-carbonyl)-N'-(4-isopropylphenyl)-N'-methylguanidine;
N-(pyridin-3-carbonyl)-N'-(1-naphthyl)guanidine;
N-(pyridin-3-carbonyl)-N'-(4-benzyloxyphenyl)guanidine;
N-(pyridin-3-carbonyl)-N'-(4-isopropylphenyl)guanidine;
N-(pyridin-3-carbonyl)-N'-(4-tert-butylphenyl)guanidine;
N-(4-methoxybenzoyl)-N'-(4-benzyloxyphenyl)guanidine;
N-(4-methoxybenzoyl)-N'-(4-isopropylphenyl)guanidine;
N-(4-methoxybenzoyl)-N'-(4-isopropoxyphenyl)guanidine;
N-(4-methoxybenzoyl)-N'-(3,4,5-trimethoxyphenyl)guanidine;
N-(1-naphthoyl)-N'-(4-benzyloxyphenyl)guanidine;
N-(1-naphthoyl)-N'-(4-isopropylphenyl)guanidine;
N-(1-naphthoyl)-N'-(4-isopropoxyphenyl)guanidine;
N-(3,4,5-trimethoxybenzoyl)-N'-(2-isopropylphenyl)guanidine;
N-(3,4,5-trimethoxybenzoyl)-N'-(4-isopropoxyphenyl)guanidine;
N-(4-butoxybenzoyl)-N'-(2-isopropylphenyl)guanidine;
N-(4-butoxybenzoyl)-N'-(4-isopropoxyphenyl)guanidine;
N-(4-butoxybenzoyl)-N'-(3,4,5-trimethoxyphenyl)guanidine;
N-(4-ethoxybenzoyl)-N'-(2-isopropylphenyl)guanidine;
N-(4-ethoxybenzoyl)-N'-(4-isopropoxyphenyl)guanidine;
N-(4-methylbenzoyl)-N'-(benzyl)guanidine;
N-(4-methylbenzoyl)-N'-(2-phenethyl)guanidine;
N-(4-methylbenzoyl)-N'-(4-phenylbutyl)guanidine;
N-(4-methylbenzoyl)-N'-(3-phenylpropyl)guanidine;
N-(4-methylbenzoyl)-N'-(1-naphthylmethyl)guanidine;
N-(4-methylbenzoyl)-N'-(2-(4-chlorophenyl)ethyl)guanidine;
N-(4-methylbenzoyl)-N'-(5-phenylpentyl)guanidine;
N-(4-methylbenzoyl)-N'-(3-phenoxypropyl)guanidine;
N-(3,4-dichlorobenzoyl)-N'-(benzyl)guanidine;
N-(3,4-dichlorobenzoyl)-N'-(3-phenylpropyl)guanidine;
N-(4-chlorobenzoyl)-N'-(2-phenethyl)guanidine;
N-(4-chlorobenzoyl)-N'-(4-phenylbutyl)guanidine;
N-(4-methoxybenzoyl)-N'-(2-phenethyl)guanidine;
N-(4-methoxybenzoyl)-N'-(4-phenylbutyl)guanidine;
N-(4-methoxybenzoyl)-N'-(2-(4-chlorophenylethyl)guanidine;
N-(4-methoxybenzoyl)-N'-(1-naphthylmethyl)guanidine;
N-(4-methoxybenzoyl)-N'-(3,4,5-trimethoxybenzyl)guanidine;
N-(4-ethoxybenzoyl)-N'-(4-phenylbutyl)guanidine;
N-(4-ethoxybenzoyl)-N'-(2-phenethyl)guanidine;
N-(4-ethoxybenzoyl)-N'-(2-(4-chlorophenyl)ethyl)guanidine;
N-(4-ethoxybenzoyl)-N'-(3-phenylpropyl)guanidine;
N-(4-ethoxybenzoyl)-N'-(1-naphthylmethyl)guanidine;
N-(4-butoxybenzoyl)-N'-(4-phenylbutyl)guanidine;
N-(4-butoxybenzoyl)-N'-(2-phenethyl)guanidine;
N-(4-butoxybenzoyl)-N'-(2-(4-chlorophenyl)ethyl)guanidine;
N-(4-butoxybenzoyl)-N'-(3-phenylpropyl)guanidine;
N-(4-butoxybenzoyl)-N'-(2-(3-indole)ethyl)guanidine;
N-(3,4,5-trimethoxybenzoyl)-N'-(4-phenylbutyl)guanidine;
N-(3,4,5-trimethoxybenzoyl)-N'-(2-(3-indole)ethyl)guanidine;
N-(3,4,5-trimethoxybenzoyl)-N'-(2-phenylethyl)guanidine;
N-(1-naphthoyl)-N'-(benzyl)guanidine;
N-(1-naphthoyl)-N'-(2-phenylethyl)guanidine;
N-(1-naphthoyl)-N'-(4-phenylbutyl)guanidine;
N-(thiophen-2-carbonyl)-N'-(benzyl)guanidine;
N-(thiophen-2-carbonyl)-N'-(2-phenylethyl)guanidine;
N-(thiophen-2-carbonyl)-N'-(4-phenylbutyl)guanidine;
N-(4-methylbenzoyl)-N'-(cyclohexyl)-N"-methylguanidine;
N-(4-methylbenzoyl)-N'-(4-phenylbutyl)-N"-methylguanidine;
N-(4-methoxybenzoyl)-N'-(5-phenylpentyl)guanidine;
N-(2-methylbenzoyl)-N'-(4-phenylbutyl)guanidine;
N-(2-methylbenzoyl)-N'-(2-isopropylphenyl)guanidine;
N-(2-methylbenzoyl)-N'-(4-isopropylphenyl)guanidine;
N-(2-methylbenzoyl)-N'-(3-phenylpropyl)guanidine;
N-(4-methoxybenzoyl)-N'-(2-phenoxypropyl)guanidine;
N-(4-butoxybenzoyl)-N'-(5-phenylpentyl)guanidine;
N-(4-methylbenzoyl)-N'-(2-phenoxyethyl)guanidine;
N-(4-methoxybenzoyl)-N'-(2-phenoxyethyl)guanidine;
N-(4-ethoxybenzoyl)-N'-[(2-benzylthio)ethyl]guanidine;
N-(4-ethoxybenzoyl)-N'- (3,4,5-trimethoxyphenyl)guanidine;
or a pharmaceutically acceptable salt of any of said compounds.

2. Use of a compound of claim 1 for the preparation of a pharmaceutical composition for use in treating a nerve degeneration disease in a mammal.

3. Use of a compound of claim 1 for the preparation of a pharmaceutical composition for use in treating a neurodegenerative disease in a mammal.

4. Use of a compound of claim 1 for the preparation of a pharmaceutical composition for use in treating Alzheimer's disease, Parkinson's disease, Huntington's disease, Amyotrophic Lateral Sclerosis, Down's Syndrome or Korsakoffs disease, Cerebral Palsy, or epilepsy in a mammal.

5. Use of a compound of claim 1 for the preparation of a pharmaceutical composition for use in treating or preventing nerve cell death or degeneration in a mammal.

6. The use of claim 5 wherein the nerve cell death or degeneration is associated with hypoxia, hypoglycemia, brain or spinal cord ischemia, retinal ischemia or brain or spinal cord trauma.

7. Use of a compound of claim 1 for the preparation of a pharmaceutical composition for use in treating a mammal suffering from or susceptible to stroke or heart attack.

8. Use of a compound of claim 1 for the preparation of a pharmaceutical composition for use in treating a mammal suffering from or susceptible to brain or spinal cord trauma.

9. Use of a compound of claim 1 for the preparation of a pharmaceutical composition for use in treating a mammal suffering from or susceptible to pain including chronic pain or neuropathic pain, peripheral necropathy, migraines, shingles, emesis, narcotic withdrawal symptoms or age-dependent dementia.

10. Use of a compound of claim 1 for the preparation of a pharmaceutical composition for use in treating a mammal suffering from or susceptible decreased blood flow or nutrient supply to retinal tissue or optic nerve, or retinal ischemia or trauma, or optic nerve injury, or glucoma.

11. Use of a compound of claim 1 for the preparation of a pharmaceutical composition for use in treating a mammal suffering from or susceptible to post-surgical neurological deficits or neurological deficits associated with cardiac arrest.

12. Use of an effective amount of an aminoglycoside antibiotic and a compound of claim 1 for the preparation of a pharmaceutical composition for use in treating an infection in a mammal.

13. The use of claim 12 wherein infection is of a Gram negative bacteria or a Gram positive bacteria.

14. The use of any one of claims 2-13 wherein the mammal is a human.

15. A pharmaceutical composition comprising a therapeutically effective amount of one or more compounds of claim 1 and a pharmaceutically acceptable carrier.

## Patentansprüche

1. Verbindung, ausgewählt aus:
N-(4-Methylbenzoyl)-N'-Methyl-N'-(3-Methylthiophenyl)guanidin;
N-(4-Methylbenzoyl)-N'-Methyl-N'-(3-Iodphenyl)guanidin;
N-(4-Methylbenzoyl)-N'-(1-Naphthyl)guanidin;
N-(4-Methylbenzoyl)-N'-(4-Benzyloxyphenyl)guanidin;
N-(4-Methylbenzoyl)-N'-(4-tertbutylphenyl)guanidin;
N-(4-Methylbenzoyl)-N'-(4-Isopropylphenyl)guanidin;
N-(4-Methylbenzoyl)-N'-(2,5-Dibromphenyl)guanidin;
N-(4-Methylbenzoyl)-N'-(4-Isopropoxyphenyl)guanidin;
N-(4-Methylbenzoyl)-N'-(3,4,5-Trimethoxyphenyl)guanidin;
N-(4-Methylbenzoyl)-N'-(2-Isopropylphenyl)guanidin;
N-(2,5-Dichtorbenzoyl)-N'-Methyl-N'-(3-Iodphenyl)guanidin;
N-(2,5-Dichlorbenzoyl)-N'-Methyl-N'-(3-Methylthiophenyl)guanidin;
N-(2,5-Dichlorbenzoyl)-N'-(1-Naphthyl)guanidin;
N-(2,5-Dichlorbenzoyl)-N'-(4-Benzyloxyphenyl)guanidin;
N-(2,5-Dichlorbenzoyl)-N'-(4-Isopropylphenyl)guanidin;
N-(2,5-Dichlorbenzoyl)-N'-(4-tertbutylphenyl)guanidin;
N-(2,5-Dichlorbenzoyl)-N'-Methyl-N'-(4-Isopropylphenyl)guanidin;
N-(Phenylacetyl)-N'-(4-Benzyloxyphenyl)guanidin;
N-(Phenylacetyl)-N'-(4-Isopropylphenyl)guanidin;
N-(Phenylacetyl)-N'-(4-tert-Butylphenyl)guanidin;
N-(Phenylacetyl)-N'-(4-Isopropoxyphenyl)guanidin;
N-(Phenylacetyl)-N'-(4-Isopropylphenyl)-N'-Methylguanidin;
N-(Adamantan-1-Carbonyl)-N'-Methyl-N'-(3-Iodphenyl)guanidin;
N-(Adamantan-1-Carbonyl)-N'-(1-Naphthyl)guanidin;
N-(Adamantan-1-Carbonyl)-N'-(4-Benzyloxyphenyl)guanidin;
N-(Adamantan-1-Carbonyl)-N'-(4-Isopropylphenyl)guanidin;
N-(Adamantan-1-Carbonyl)-N'-(4-tert-Butylphenyl)guanidin;
N-(Adamantan-1-Carbonyl)-N'-(2,5-Dibromphenyl)guanidin;
N-(Adamantan-1-Carbonyl)-N'-(4-Isopropylphenyl)-N'-Methyl)guanidin;
N-(4-Chlorbenzoyl)-N'-Methyl-N'-(3-Iodphenyl)guanidin;
N-(4-Chlorbenzoyl)-N'-Methyl-N'-(3-Methylthiophenyl)guanidin;
N-(4-Chlorbenzoyl)-N'-(1-Naphthyl)guanidin;
N-(4-Chlorbenzoyl)-N'-(4-Benzyloxyphenyl)guanidin;
N-(4-Chlorbenzoyl)-N'-(4-Isopropylphenyl)guanidin;
N-(4-Chlorbenzoyl)-N'-(4-tert-Butylphenyl)guanidin;
N-(4-Chlorbenzoyl)-N'-(2,5-Dibromphenyl)guanidin;
N-(3,4-Dichlorbenzoyl)-N'-Methyl-N'-(3-Iodphenyl)guanidin;
N-(3,4-Dichlorbenzoyl)-N'-(1-Naphthyl)guanidin;
N-(3,4-Dichlorbenzoyl)-N'-(4-Benzyloxyphenyl)guanidin;
N-(3,4-Dichlorbenzoyl)-N'-(4-Isopropylphenyl)guanidin;
N-(3,4-Dichlorbenzoyl)-N'-(4-tert-Butylphenyl)guanidin;
N-(3,4-Dichlorbenzoyl)-N'-Methyl-N'-(4-Isopropylphenyl)guanidin;
N-(Thiophen-2-Carbonyl)-N'-Methyl-N'-(3-Iodphenyl)guanidin;
N-(Thiophen-2-Carbonyl)-N'-Methyl-N'-(3-Methylthiophenyl)guanidin;
N-(Thiophen-2-Carbonyl)-N'-(1-Naphthyl)guanidin;
N-(Thiophen-2-Carbonyl)-N'-(4-Benzyloxyphenyl)guanidin;
N-(Thiophen-2-Carbonyl)-N'-(4-Isopropylphenyl)guanidin;
N-(Thiophen-2-Carbonyl)-N'-(4-tert-Butylphenyl)guanidin;
N-(Thiophen-2-Carbonyl)-N'-Methyl-N'-(4-Isopropylphenyl)guanidin;
N-(Furan-2-Carbonyl)-N'-Methyl-N'-(3-Iodphenyl)guanidin;
N-(Furan-2-Carbonyl)-N'-Methyl-N'-(3-Methylthiophenyl)guanidin;
N-(Furan-2-Carbonyl)-N'-(1-Naphthyl)guanidin;
N-(Furan-2-Carbonyl)-N'-(4-Benzyloxyphenyl)guanidin;
N-(Furan-2-Carbonyl)-N'-(4-Isopropylphenyl)guanidin;.
N-(Furan-2-Carbonyl)-N'-(4-tert-Butylphenyl)guanidin;
N-(Furan-2-Carbonyl)-N'-(4-Isopropylphenyl)-N'-Methylguanidin;
N-(Pyridin-3-Carbonyl)-N'-(1-Naphthyl)guanidin;
N-(Pyridin-3-Carbonyl)-N'-(4-Benzyloxyphenyl)guanidin;
N-(Pyridin-3-Carbonyl)-N'-(4-Isopropylphenyl)guanidin;
N-(Pyridin-3-Carbonyl)-N'-(4-tert-Butylphenyl)guanidin;
N-(4-Methoxybenzoyl)-N'-(4-Benzyloxyphenyl)guanidin;
N-(4-Methoxybenzoyl)-N'-(4-Isopropylphenyl)guanidin;
N-(4-Methoxybenzoyl)-N'-(4-Isopropoxyphenyl)guanidin;
N-(4-Methoxybenzoyl)-N'-(3,4,5-Trimethoxyphenyl)guanidin;
N-(1-Naphthoyl)-N'-(4-Benzyloxyphenyl)guanidin;
N-(1-Naphthoyl)-N'-(4-Isopropylphenyl)guanidin;
N-(1-Naphthoyl)-N'-(4-Isopropoxyphenyl)guanidin;
N-(3,4,5-Trimethoxybenzoyl)-N'-(2-Isopropylphenyl)guanidin;
N-(3,4,5-Trimethoxybenzoyl)-N'-(4-Isopropoxyphenyl)guanidin;
N-(4-Butoxybenzoyl)-N'-(2-Isopropylphenyl)guanidin;
N-(4-Butoxybenzoyl)-N'-(4-Isopropoxyphenyl)guanidin;
N-(4-Butoxybenzoyl)-N'-(3,4,5-Trimethoxyphenyl)guanidin;
N-(4-Ethoxybenzoyl)-N'-(2-Isopropylphenyl)guanidin;
N-(4-Ethoxybenzoyl)-N'-(4-Isopropoxyphenyl)guanidin;
N-(4-Methylbenzoyl)-N'-(Benzyl)guanidin;
N-(4-Methylbenzoyl)-N'-(2-Phenethyl)guanidin;
N-(4-Methylbenzoyl)-N'-(4-Phenylbutyl)guanidin;
N-(4-Methylbenzoyl)-N'-(3-Phenylpropyl)guanidin;
N-(4-Methylbenzoyl)-N'-(1-Naphthylmethyl)guanidin;
N-(4-Methylbenzoyl)-N'-(2-(4-Chlorphenyl)ethyl)guanidin;
N-(4-Methylbenzoyl)-N'-(5-Phenylpentyl)guanidin;
N-(4-Methylbenzoyl)-N'-(3-Phenoxypropyl)guanidin;
N-(3,4-Dichlorbenzoyl)-N'-(Benzyl)guanidin;
N-(3,4-Dichlorbenzoyl)-N'-(3-Phenylpropyl)guanidin;
N-(4-Chlorbenzoyl)-N'-(2-Phenethyl)guanidin;
N-(4-Chlorbenzoyl)-N'-(4-Phenylbutyl)guanidin;
N-(4-Methoxybenzoyl)-N'-(2-Phenethyl)guanidin;
N-(4-Methoxybenzoyl)-N'-(4-Phenylbutyl)guanidin;
N-(4-Methoxybenzoyl)-N'-(2-(4-Chlorphenylethyl)guanidin;
N-(4-Methoxybenzoyl)-N'-(1-Naphthylmethyl)guanidin;
N-(4-Methoxybenzoyl)-N'-(3,4,5-Trimethoxybenzyl)guanidin;
N-(4-Ethoxybenzoyl)-N'-(4-Phenylbutyl)guanidin;
N-(4-Ethoxybenzoyl)-N'-(2-Phenethyl)guanidin;
N-(4-Ethoxybenzoyl)-N'-(2-(4-Chlorphenyl)ethyl)guanidin;
N-(4-Ethoxybenzoyl)-N'-(3-Phenylpropyl)guanidin;
N-(4-Ethoxybenzoyl)-N'-(1-Naphthylmethyl)guanidin;
N-(4-Butoxybenzoyl)-N'-(4-Phenylbutyl)guanidin;
N-(4-Butoxybenzoyl)-N'-(2-Phenethyl)guanidin;
N-(4-Butoxybenzoyl)-N'-(2-(4-Chlorphenyl)ethyl)guanidin;
N-(4-Butoxybenzoyl)-N'-(3-Phenylpropyl)guanidin;
N-(4-Butoxybenzoyl)-N'-(2-(3-Indol)ethyl)guanidin;
N-(3,4,5-Trimethoxybenzoyl)-N'-(4-Phenylbutyl)guanidin;
N-(3,4,5-Trimethoxybenzoyl)-N'-(2-(3-Indol)ethyl)guanidin;
N-(3,4,5-Trimethoxybenzoyl)-N'-(2-Phenylethyl)guanidin;
N-(1-Naphthoyl)-N'-(Benzyl)guanidin;
N-(1-Naphthoyl)-N'-(2-Phenylethyl)guanidin;
N-(1-Naphthoyl)-N'-(4-Phenylbutyl)guanidin;
N-(Thiophen-2-Carbonyl)-N'-(Benzyl)guanidin;
N-(Thiophen-2-Carbonyl)-N'-(2-Phenylethyl)guanidin;
N-(Thiophen-2-Carbonyl)-N'-(4-Phenylbutyl)guanidin;
N-(4-Methylbenzoyl)-N'-(Cyclohexyl)-N"-Methylguanidin;
N-(4-Methylbenzoyl)-N'-(4-Phenylbutyl)-N"-Methylguanidin;
N-(4-Methoxybenzoyl)-N'-(5-Phenylpentyl)guanidin;
N-(2-Methylbenzoyl)-N'-(4-Phenylbutyl)guanidin;
N-(2-Methylbenzoyl)-N'-(2-Isopropylphenyl)guanidin;
N-(2-Methylbenzoyl)-N'-(4-Isopropylphenyl)guanidin;
N-(2-Methylbenzoyl)-N'-(3-Phenylpropyl)guanidin;
N-(4-Methoxybenzoyl)-N'-(2-Phenoxypropyl)guanidin;
N-(4-Butoxybenzoyl)-N'-(5-Phenylpentyl)guanidin;
N-(4-Methylbenzoyl)-N'-(2-Phenoxyethyl)guanidin;
N-(4-Methoxybenzoyl)-N'-(2-Phenoxyethyl)guanidin;
N-(4-Ethoxybenzoyl)-N'-[(2-Benzylthio)ethyl]guanidin;
N-(4-Ethoxybenzoyl)-N'-(3,4,5-Trimethoxyphenyl)guanidin;
oder einem pharmazeutisch verträglichen Salz von irgendeinem der Verbindungen.

2. Anwendung einer Verbindung nach Anspruch 1 zur Herstellung einer pharmazeutischen Zusammensetzung zur Anwendung bei der Behandlung einer Nervendegenerationserkrankung bei einem Säugetier.

3. Anwendung einer Verbindung nach Anspruch 1 zur Herstellung einer pharmazeutischen Zusammensetzung zur Anwendung bei der Behandlung einer neurodegenerativen Erkrankung bei einem Säugetier.

4. Anwendung einer Verbindung nach Anspruch 1 zur Herstellung einer pharmazeutischen Zusammensetzung zur Anwendung bei der Behandlung von Alzheimerkrankheit, Parkinsonkrankheit, Huntingtonkrankheit, amyotropher Lateralsklerose, Down-Syndrom oder Korsakoff-Krankheit, zerebraler Lähmung oder Epilepsie bei einem Säugetier.

5. Anwendung einer Verbindung nach Anspruch 1 zur Herstellung einer pharmazeutischen Zusammensetzung zur Anwendung bei der Behandlung oder Vorbeugung von Nervenzellentod oder -degeneration bei einem Säugetier.

6. Anwendung nach Anspruch 5, worin der Nervenzellentod oder die Nervenzellendegeneration mit Hypoxie, Hypoglykämie, Gehirn- oder Rückenmarkischämie, Netzhautischämie oder Gehirn- oder Rückenmarktrauma verbunden ist.

7. Anwendung einer Verbindung nach Anspruch 1 zur Herstellung einer pharmazeutischen Zusammensetzung zur Anwendung bei der Behandlung eines Säugetiers, das an Schlaganfall oder Herzinfakt leidet oder dafür anfällig ist.

8. Anwendung einer Verbindung nach Anspruch 1 zur Herstellung einer pharmazeutischen Zusammensetzung zur Anwendung bei der Behandlung eines Säugetiers, das an Gehirn- oder Rückenmarktrauma leidet oder dafür anfällig ist.

9. Anwendung einer Verbindung nach Anspruch 1 zur Herstellung einer pharmazeutischen Zusammensetzung zur Anwendung bei der Behandlung eines Säugetiers, das an Schmerz, hierunter kronischem Schmerz oder neuropathischem Schmerz, peripherer Nekropathie, Migränen, Gürtelrose, Erbrechen, Suchtgift-Entzugssymptomen oder altersabhängiger Demenz leidet oder dafür anfällig ist.

10. Anwendung einer Verbindung nach Anspruch 1 zur Herstellung einer pharmazeutischen Zusammensetzung zur Anwendung bei der Behandlung eines Säugetiers, das an reduziertem Blutfluss oder reduzierter Nährstoffversorgung zum Netzhautgewebe oder zum Sehnerv, oder Netzhautischämie oder -trauma, oder Sehnervschädigung oder Glucoma leidet oder dafür anfällig ist.

11. Anwendung einer Verbindung nach Anspruch 1 zur Herstellung einer pharmazeutischen Zusammensetzung zur Anwendung bei der Behandlung eines Säugetiers, das an post-chirurgischen neurologischen Defiziten oder neurologischen Defiziten, die mit Herzstillstand verbunden sind, leidet oder dafür anfällig ist.

12. Anwendung einer effektiven Menge eines Aminoglykosid-Antibiotikums und einer Verbindung nach Anspruch 1 zur Herstellung einer pharmazeutischen Zusammensetzung zur Anwendung bei der Behandlung einer Infektion bei einem Säugetier.

13. Anwendung nach Anspruch 12, worin die Infektion von einer Gramnegativen Bakterie oder Gram-positiven Bakterie ist.

14. Anwendung nach irgendeinem der Ansprüche 2-13, worin das Säugetier ein Mensch ist.

15. Pharmazeutische Zusammensetzung, umfassend eine therapeutisch effektive Menge von einer oder mehreren Verbindungen nach Anspruch 1 und einen pharmazeutisch verträglichen Träger.

## Revendications

1. Un composé choisi parmi :
la N-(4-méthylebenzoyle)-N'-méthyle-N'-(3-méthylethiophényle)guanidine ;
la N-(4-méthylebenzoyle)-N'-méthyle-N'-(3-iodophényle)guanidine ;
la N-(4-méthylebenzoyle)-N'-(1-naphtyle)guanidine ;
la N-(4-méthylebenzoyle)-N'-(4-benzyloxyphényle)guanidine ;
la N-(4-méthylebenzoyle)-N'-(4-tert-butylephényle)guanidine ;
la N-(4-méthylebenzoyle)-N'-(4-isopropylephényle)guanidine ;
la N-(4-méthylebenzoyle)-N'-(2,5-dibromophényle)guanidine ;
la N-(4-méthylebenzoyle)-N'-(4-isopropoxyphényle)guanidine ;
la N-(4-méthylebenzoyle)-N'-(3,4,5-triméthoxyphényle)guanidine ;
la N-(4-méthylebenzoyle)-N'-(2-isopropylephényle)guanidine ;
la N-(2,5-dichlorobenzoyle)-N'-méthyle-N'-(3-iodophényte)guanidine ;
la N-(2,5-dichlorobenzoyle)-N'-méthyle-N'-(3-méthylethiophényle)guanidine ;
la N-(2,5-dichlorobenzoyle)-N'-(1-naphtyle)guanidine ;
la N-(2,5-dichlorobenzoyle)-N'-(4-benzyloxyphényle)guanidine ;
la N-(2,5-dichlorobenzoyle)-N'-(4-isopropylephényle)guanidine ;
la N-(2,5-dichlorobenzoyle)-N'-(4-tert-butylephényle)guanidine ;
la N-(2,5-dichlorobenzoyle)-N'-méthyle-N'-(4-isopropylephényle)guanidine ;
la N-(phényleacétyle)-N'-(4-benzyloxyphényle)guanidine ;
la N-(phényleacétyle)-N'-(4-isopropylephényle)guanidine ;
la N-(phényleacétyle)-N'-(4-tert-butylephényle)guanidine ;
la N-(phényleacétyle)-N'-(4-isopropoxyphényle)guanidine ;
la N-(phényleacétyle)-N'-(4-isopropylephényle)-N'-méthyleguanidine ;
la N-(adamantane-1-carbonyle)-N'-méthyle-N'-(3-iodophényle)guanidine ;
la N-(adamantane-1-carbonyle)-N'-(1-naphtyle)guanidine ;
la N-(adamantane-1-carbonyle)-N'-(4-benzyloxyphényle)guanidine ;
la N-(adamantane-1-carbonyle)-N'-(4-isopropylephényle)guanidine ;
la N-(adamantane-1-carbonyle)-N'-(4-tert-butylephényle)guanidine ;
la N-(adamantane-1-carbonyle)-N'-(2,5-dibromophényle)guanidine ;
la N-(adamantane-1-carbonyle)-N'-(4-isopropylephényle)-N'-méthyleguanidine ;
la N-(4-chlorobenzoyle)-N'-méthyle-N'-(3-iodophényle)guanidine ;
la N-(4-chlorobenzoyle)-N'-méthyle-N'-(3-méthylethiophényle)guanidine ;
la N-(4-chlorobenzoyle)-N'-(1-naphtyle)guanidine ;
la N-(4-chlorobenzoyle)-N'-(4-benzyloxyphényle)guanidine ;
la N-(4-chlorobenzoyle)-N'-(4-isopropylephényle)guanidine ;
la N-(4-chlorobenzoyle)-N'-(4-tert-butylephényle)guanidine ;
la N-(4-chlorobenzoyle)-N'-(2,5-dibromophényle)guanidine ;
la N-(3,4-dichlorobenzoyle)-N'-méthyle-N'-(3-iodophényle)guanidine ;
la N-(3,4-dichlorobenzoyle)-N'-(1-naphtyle)guanidine ;
la N-(3,4-dichlorobenzoyle)-N'-(4-benzyloxyphényle)guanidine ;
la N-(3,4-dichlorobenzoyle)-N'-(4-isopropylephényle)guanidine ;
la N-(3,4-dichlorobenzoyle)-N'-(4-tert-butylephényle)guanidine ;
la N-(3,4-dichlorobenzoyle)-N'-méthyle-N'-(4-isopropylephényle)guanidine ;
la N-(thiophène-2-carbonyle)-N'-méthyle-N'-(3-iodophényle)guanidine ;
la N-(thiophène-2-carbonyle)-N'-méthyle-N'-(3-méthylethiophényle)guanidine ;
la N-(thiophène-2-carbonyle)-N'-(1-naphtyle)guanidine ;
la N-(thiophène-2-carbonyle)-N'-(4-benzyloxyphényle)guanidine ;
la N-(thiophène-2-carbonyle)-N'-(4-isopropylephényle)guanidine ;
la N-(thiophène-2-carbonyle)-N'-(4-tert-butylephényle)guanidine ;
la N-(thiophène-2-carbonyle)-N'-méthyle-N'-(4-isopropylephényle)guanidine ;
la N-(furane-2-carbonyle)-N'-méthyle-N'-(3-iodophényle)guanidine ;
la N-(furane-2-carbonyle)-N'-méthyle-N'-(3-méthylethiophényle)guanidine ;
la N-(furane-2-carbonyle)-N'-(1-naphtyle)guanidine ;
la N-(furane-2-carbonyle)-N'-(4-benzyloxyphényle)guanidine ;
la N-(furane-2-carbonyle)-N'-(4-isopropylephényle)guanidine ;
la N-(furane-2-carbonyle)-N'-(4-tert-butylephényle)guanidine ;
la N-(furane-2-carbonyle)-N'-(4-isopropylephényle)-N'-méthyleguanidine ;
la N-(pyridine-3-carbonyle)-N'-(1-naphtyle)guanidine ;
la N-(pyridine-3-carbonyle)-N'-(4-benzyloxyphényle)guanidine ;
la N-(pyridine-3-carbonyle)-N'-(4-isopropylephényle)guanidine ;
la N-(pyridine-3-carbonyle)-N'-(4-tert-butylephényle)guanidine ;
la N-(4-méthoxybenzoyle)-N'-(4-benzyloxyphényle)guanidine ;
la N-(4-méthoxybenzoyle)-N'-(4-isopropylephényle)guanidine ;
la N-(4-méthoxybenzoyle)-N'-(4-isopropoxyphényle)guanidine ;
la N-(4-méthoxybenzoyle)-N'-(3,4,5-triméthoxyphényle)guanidine ;
la N-(1-naphthoyle)-N'-(4-benzyloxyphényle)guanidine ;
la N-(1-naphthoyle)-N'-(4-isopropylephényle)guanidine ;
la N-(1-naphthoyle)-N'-(4-isopropoxyphényle)guanidine ;
la N-(3,4,5-triméthoxybenzoyle)-N'-(2-isopropylephényle)guanidine ;
la N-(3,4,5-triméthoxybenzoyle)-N'-(4-isopropoxyphényle)guanidine ;
la N-(4-butoxybenzoyle)-N'-(2-isopropylephényle)guanidine ;
la N-(4-butoxybenzoyle)-N'-(4-isopropoxyphényle)guanidine ;
la N-(4-butoxybenzoyle)-N'-(3,4,5-triméthoxyphényle)guanidine ;
la N-(4-éthoxybenzoyle)-N'-(2-isopropylephényle)guanidine ;
la N-(4-éthoxybenzoyle)-N'-(4-isopropoxyphényle)guanidine ;
la N-(4-méthylebenzoyle)-N'-(benzyle)guanidine ;
la N-(4-méthylebenzoyle)-N'-(2-phénéthyle)guanidine ;
la N-(4-méthylebenzoyle)-N'-(4-phénylebutyle)guanidine ;
la N-(4-méthylebenzoyle)-N'-(3-phénylepropyle)guanidine ;
la N-(4-méthylebenzoyle)-N'-(1-naphtyleméthyle)guanidine ;
la N-(4-méthylebenzoyle)-N'-(2-(4-chlorophényle)éthyle)guanidine ;
la N-(4-méthylebenzoyle)-N'-(5-phénylepentyle)guanidine ;
la N-(4-méthylebenzoyle)-N'-(3-phénoxypropyle)guanidine ;
la N-(3,4-dichlorobenzoyle)-N'-(benzyle)guanidine ;
la N-(3,4-dichlorobenzoyle)-N'-(3-phénylepropyle)guanidine ;
la N-(4-chlorobenzoyle)-N'-(2-phénéthyle)guanidine ;
la N-(4-chlorobenzoyle)-N'-(4-phénylebutyle)guanidine ;
la N-(4-méthoxybenzoyle)-N'-(2-phénéthyle)guanidine ;
la N-(4-méthoxybenzoyle)-N'-(4-phénylebutyle)guanidine ;
la N-(4-méthoxybenzoyle)-N'-(2-(4-chlorophényle)éthyle)guanidine ;
la N-(4-méthoxybenzoyle)-N'-(1-naphtyleméthyle)guanidine ;
la N-(4-méthoxybenzoyle)-N'-(3,4,5-triméthoxybenzyle)guanidine ;
la N-(4-éthoxybenzoyle)-N'-(4-phénylebutyle)guanidine ;
la N-(4-éthoxybenzoyle)-N'-(2-phénéthyle)guanidine ;
la N-(4-éthoxybenzoyle)-N'-(2-(4-chlorophényle)éthyle)guanidine ;
la N-(4-éthoxybenzoyle)-N'-(3-phénylepropyle)guanidine ;
la N-(4-éthoxybenzoyle)-N'-(1-naphtyleméthyle)guanidine ;
la N-(4-butoxybenzoyle)-N'-(4-phénylebutyle)guanidine ;
la N-(4-butoxybenzoyle)-N'-(2-phénéthyle)guanidine ;
la N-(4-butoxybenzoyle)-N'-(2-(4-chlorophényle)éthyle)guanidine ;
la N-(4-butoxybenzoyle)-N'-(3-phénylepropyle)guanidine ;
la N-(4-butoxybenzoyle)-N'-(2-(3-indole)éthyle)guanidine ;
la N-(3,4,5-triméthoxybenzoyle)-N'-(4-phénylebutyle)guanidine ;
la N-(3,4,5-triméthoxybenzoyle)-N'-(2-(3-indole)éthyle)guanidine ;
la N-(3,4,5-triméthoxybenzoyle)-N'-(2-phényleéthyle)guanidine ;
la N-(1-naphthoyle)-N'-(benzyle)guanidine ;
la N-(1-naphthoyle)-N'-(2-phényleéthyle)guanidine ;
la N-(1-naphthoyle)-N'-(4-phénylebutyle)guanidine ;
la N-(thiophène-2-carbonyle)-N'-(benzyle)guanidine ;
la N-(thiophène-2-carbonyle)-N'-(2-phényleéthyle)guanidine ;
la N-(thiophène-2-carbonyle)-N'-(4-phénylebutyle)guanidine ;
la N-(4-méthylebenzoyle)-N'-(cyclohéxyle)-N"-méthyleguanidine ;
la N-(4-méthylebenzoyle)-N'-(4-phénylebutyle)-N"-méthyleguanidine ;
la N-(4-méthoxybenzoyle)-N'-(5-phénylepentyle)guanidine ;
la N-(2-méthylebenzoyle)-N'-(4-phénylebutyle)guanidine ;
la N-(2-méthylebenzoyle)-N'-(2-isopropylephényle)guanidine ;
la N-(2-méthylebenzoyle)-N'-(4-isopropylephényle)guanidine ;
la N-(2-méthylebenzoyle)-N'-(3-phénylepropyle)guanidine ;
la N-(4-méthoxybenzoyle)-N'-(2-phénoxypropyle)guanidine ;
la N-(4-butoxybenzoyle)-N'-(5-phénylepentyle)guanidine ;
la N-(4-méthylebenzoyle)-N'-(2-phénoxyéthyle)guanidine ;
la N-(4-méthoxybenzoyle)-N'-(2-phénoxyéthyle)guanidine ;
la N-(4-éthoxybenzoyle)-N'-[(2-benzylethio)éthyle]guanidine ;
la N-(4-éthoxybenzoyle)-N'-(3,4,5-triméthoxyphényle)guanidine ;
ou un sel pharmaceutiquement acceptable de l'un desdits composés.

2. Utilisation d'un composé selon la revendication 1 pour la préparation d'une composition pharmaceutique destinée au traitement d'une maladie de dégénérescence nerveuse chez un mammifère.

3. Utilisation d'un composé selon la revendication 1 pour la préparation d'une composition pharmaceutique destinée au traitement d'une maladie neuro-dégénérative chez un mammifère.

4. Utilisation d'un composé selon la revendication 1 pour la préparation d'une composition pharmaceutique destinée au traitement de la maladie d'Alzheimer, de la maladie de Parkinson, de la maladie de Huntington, de la sclérose latérale amyotrophique, du syndrome de Down ou bien de la maladie de Korsakoff, de l'infirmité motrice cérébrale ou de l'épilepsie chez un mammifère.

5. Utilisation d'un composé selon la revendication 1 pour la préparation d'une composition pharmaceutique destinée au traitement ou à la prévention de la mort ou de la dégénérescence de cellules nerveuses chez un mammifère.

6. L'utilisation selon la revendication 5 dans laquelle la mort ou la dégénérescence de cellules nerveuses est associée à l'hypoxie, à l'ischémie du cerveau ou de la moelle épinière, à l'ischémie rétinienne ou à un traumatisme du cerveau ou de la moelle épinière.

7. Utilisation d'un composé selon la revendication 1 pour la préparation d'une composition pharmaceutique destinée au traitement d'un mammifère atteint de ou prédisposé aux accidents vasculaires ou aux infarctus du myocarde.

8. Utilisation d'un composé selon la revendication 1 pour la préparation d'une composition pharmaceutique destinée au traitement d'un mammifère atteint de ou prédisposé aux traumatismes du cerveau ou de la moelle épinière.

9. Utilisation d'un composé selon la revendication 1 pour la préparation d'une composition pharmaceutique destinée au traitement d'un mammifère atteint de ou prédisposé aux douleurs chroniques ou douleurs neuropathiques, aux neuropathies périphériques, aux migraines, au zona, aux vomissements, aux symptômes de sevrage de stupéfiants ou à la démence due au vieillissement.

10. Utilisation d'un composé selon la revendication 1 pour la préparation d'une composition pharmaceutique destinée au traitement d'un mammifère atteint de ou prédisposé aux réductions du flux sanguin ou de l'apport en éléments nutritifs vers le tissu rétinien ou le nerf optique, ou à l'ischémie ou aux traumatismes rétiniens, ou aux lésions au niveau du nerf optique ou à un glaucome.

11. Utilisation d'un composé selon la revendication 1 pour la préparation d'une composition pharmaceutique destinée au traitement d'un mammifère atteint de ou prédisposé aux déficiences neurologiques post-chirurgicales ou aux déficiences neurologiques associées à un arrêt cardiaque.

12. Utilisation d'un composé selon la revendication 1 pour la préparation d'une composition pharmaceutique destinée au traitement d'une infection chez un mammifère.

13. L'utilisation selon la revendication 12, dans laquelle l'infection est par une bactérie Gram négative ou par une bactérie Gram positive.

14. L'utilisation selon l'une quelconque des revendications 2 à 14, dans laquelle le mammifère est un être humain.

15. Une composition pharmaceutique comprenant une quantité thérapeutiquement efficace d'une ou de plusieurs des composés selon la revendication 1 et un véhicule pharmaceutiquement acceptable.
